# EUROPEAN PATENT APPLICATION

(11) **EP 3 906 924 A1**
(43) Date of publication of application: **10.11.2021**
(21) Application number: 20173672.5
(22) Date of filing: 08.05.2020
(51) Int. Cl.: A61K 31/4706, A61K 31/501, C12Q 1/48, G01N 33/50

(54) **MODULATION OF MIXED LINEAGE KINASE DOMAIN-LIKE PROTEIN SIGNALING**

(71) Applicant: Eberhard Karls Universität Tübingen, 72074 Tübingen (DE)
(72) Inventor: García Sáez, Ana Jesús, 72076 Tübingen (DE); Ros Quincoces, Uris Lianne, 72072 Tübingen (DE); Valiente Flores, Pedro Alberto, Toronto, Ontario M3H 2S9 (CA); Wong, Wendy Wei-Lynn, 8057 Zürich (CH); Walczak, Henning, London W4 2BS (GB); de las Nieves Peltzer, Maria, Croydon CR0 6JE (GB)
(74) Representative: Kuttenkeuler, David

(57) **Abstract**

The invention is based on a method of modulating the activation or inhibition of Mixed lineage kinase domain-like (MLKL) protein, or a MLKL variant protein, via modulating the the intramolecular interaction between the C-terminal helix (He) of the psK domain and a hydrophobic groove in the MLKL protein. The invention provides methods and compounds for to selectively target the herein firstly disclosed intramolecular interaction of MLKL protein. Based on the herein disclosed essential intramolecular rearrangement of MLKL, the invention provides small molecules capable of specifically inhibiting mouse or human MLKL. The invention provides uses, including medical applications such as treatments, of MLKL driven conditions including necroptosis, cell trafficking, pathological immune responses and/or inflammation.

SEQUENCE LISTING

<110>  Eberhard Karls Universität Tübingen

<120>  MODULATION OF MIXED LINEAGE KINASE DOMAIN-LIKE PROTEIN SIGNALING

<130>  U30959EP

<160>  4

<170>  PatentIn version 3.5

<210>  1
<211>  472
<212>  PRT
<213>  mus musculus

<400>  1

Met Asp Lys Leu Gly Gln Ile Ile Lys Leu Gly Gln Leu Ile Tyr Glu
1               5                   10                  15

Gln Cys Glu Lys Met Lys Tyr Cys Arg Lys Gln Cys Gln Arg Leu Gly
            20                  25                  30

Asn Arg Val His Gly Leu Leu Gln Pro Leu Gln Arg Leu Gln Ala Gln
        35                  40                  45

Gly Lys Lys Asn Leu Pro Asp Asp Ile Thr Ala Ala Leu Gly Arg Phe
    50                  55                  60

Asp Glu Val Leu Lys Glu Ala Asn Gln Gln Ile Glu Lys Phe Ser Lys
65                  70                  75                  80

Lys Ser His Ile Trp Lys Phe Val Ser Val Gly Asn Asp Lys Ile Leu
                85                  90                  95

Phe His Glu Val Asn Glu Lys Leu Arg Asp Val Trp Glu Glu Leu Leu
            100                 105                 110

Leu Leu Leu Gln Val Tyr His Trp Asn Thr Val Ser Asp Val Ser Gln
        115                 120                 125

Pro Ala Ser Trp Gln Gln Glu Asp Arg Gln Asp Ala Glu Glu Asp Gly
    130                 135                 140

Asn Glu Asn Met Lys Val Ile Leu Met Gln Leu Gln Ile Ser Val Glu
145                 150                 155                 160

Glu Ile Asn Lys Thr Leu Lys Gln Cys Ser Leu Lys Pro Thr Gln Glu
                165                 170                 175

Ile Pro Gln Asp Leu Gln Ile Lys Glu Ile Pro Lys Glu His Leu Gly
        180                 185                 190

Pro Pro Trp Thr Lys Leu Lys Thr Ser Lys Met Ser Thr Ile Tyr Arg
    195                 200                 205

Gly Glu Tyr His Arg Ser Pro Val Thr Ile Lys Val Phe Asn Asn Pro
210                 215                 220

Gln Ala Glu Ser Val Gly Ile Val Arg Phe Thr Phe Asn Asp Glu Ile
225                 230                 235                 240

Lys Thr Met Lys Lys Phe Asp Ser Pro Asn Ile Leu Arg Ile Phe Gly
                245                 250                 255

Ile Cys Ile Asp Gln Thr Val Lys Pro Pro Glu Phe Ser Ile Val Met
            260                 265                 270

Glu Tyr Cys Glu Leu Gly Thr Leu Arg Glu Leu Leu Asp Arg Glu Lys
        275                 280                 285

Asp Leu Thr Met Ser Val Arg Ser Leu Leu Val Leu Arg Ala Ala Arg
    290                 295                 300

Gly Leu Tyr Arg Leu His His Ser Glu Thr Leu His Arg Asn Ile Ser
305                 310                 315                 320

Ser Ser Ser Phe Leu Val Ala Gly Gly Tyr Gln Val Lys Leu Ala Gly
                325                 330                 335

Phe Glu Leu Ser Lys Thr Gln Asn Ser Ile Ser Arg Thr Ala Lys Ser
            340                 345                 350

Thr Lys Ala Glu Arg Ser Ser Ser Thr Ile Tyr Val Ser Pro Glu Arg
        355                 360                 365

Leu Lys Asn Pro Phe Cys Leu Tyr Asp Ile Lys Ala Glu Ile Tyr Ser
    370                 375                 380

Phe Gly Ile Val Leu Trp Glu Ile Ala Thr Gly Lys Ile Pro Phe Glu
385                 390                 395                 400

Gly Cys Asp Ser Lys Lys Ile Arg Glu Leu Val Ala Glu Asp Lys Lys
                405                 410                 415

Gln Glu Pro Val Gly Gln Asp Cys Pro Glu Leu Leu Arg Glu Ile Ile
            420                 425                 430

Asn Glu Cys Arg Ala His Glu Pro Ser Gln Arg Pro Ser Val Asp Gly
        435                 440                 445

Arg Ser Leu Ser Gly Arg Glu Arg Ile Leu Glu Arg Leu Ser Ala Val
    450                 455                 460

Glu Glu Ser Thr Asp Lys Lys Val
465                 470

<210>  2
<211>  464
<212>  PRT
<213>  Mus musculus

<400>  2

Met Asp Lys Leu Gly Gln Ile Ile Lys Leu Gly Gln Leu Ile Tyr Glu
1               5                   10                  15

Gln Cys Glu Lys Met Lys Tyr Cys Arg Lys Gln Cys Gln Arg Leu Gly
            20                  25                  30

Asn Arg Val His Gly Leu Leu Gln Pro Leu Gln Arg Leu Gln Ala Gln
        35                  40                  45

Gly Lys Lys Asn Leu Pro Asp Asp Ile Thr Ala Ala Leu Gly Arg Phe
    50                  55                  60

Asp Glu Val Leu Lys Glu Ala Asn Gln Gln Ile Glu Lys Phe Ser Lys
65                  70                  75                  80

Lys Ser His Ile Trp Lys Phe Val Ser Val Gly Asn Asp Lys Ile Leu
                85                  90                  95

Phe His Glu Val Asn Glu Lys Leu Arg Asp Val Trp Glu Glu Leu Leu
            100                 105                 110

Leu Leu Leu Gln Val Tyr His Trp Asn Thr Val Ser Asp Val Ser Gln
        115                 120                 125

Pro Ala Ser Trp Gln Gln Glu Asp Arg Gln Asp Ala Glu Glu Asp Gly
    130                 135                 140

Asn Glu Asn Met Lys Val Ile Leu Met Gln Leu Gln Ile Ser Val Glu
145                 150                 155                 160

Glu Ile Asn Lys Thr Leu Lys Gln Cys Ser Leu Lys Pro Thr Gln Glu
                165                 170                 175

Ile Pro Gln Asp Leu Gln Ile Lys Glu Ile Pro Lys Glu His Leu Gly
        180                 185                 190

Pro Pro Trp Thr Lys Leu Lys Thr Ser Lys Met Ser Thr Ile Tyr Arg
    195                 200                 205

Gly Glu Tyr His Arg Ser Pro Val Thr Ile Lys Val Phe Asn Asn Pro
210                 215                 220

Gln Ala Glu Ser Val Gly Ile Val Arg Phe Thr Phe Asn Asp Glu Ile
225                 230                 235                 240

Lys Thr Met Lys Lys Phe Asp Ser Pro Asn Ile Leu Arg Ile Phe Gly
                245                 250                 255

Ile Cys Ile Asp Gln Thr Val Lys Pro Pro Glu Phe Ser Ile Val Met
            260                 265                 270

Glu Tyr Cys Glu Leu Gly Thr Leu Arg Glu Leu Leu Asp Arg Glu Lys
        275                 280                 285

Asp Leu Thr Met Ser Val Arg Ser Leu Leu Val Leu Arg Ala Ala Arg
    290                 295                 300

Gly Leu Tyr Arg Leu His His Ser Glu Thr Leu His Arg Asn Ile Ser
305                 310                 315                 320

Ser Ser Ser Phe Leu Val Ala Gly Gly Tyr Gln Val Lys Leu Ala Gly
                325                 330                 335

Phe Glu Leu Ser Lys Thr Gln Asn Ser Ile Ser Arg Thr Ala Lys Ser
            340                 345                 350

Thr Lys Ala Glu Arg Ser Ser Ser Thr Ile Tyr Val Ser Pro Glu Arg
        355                 360                 365

Leu Lys Asn Pro Phe Cys Leu Tyr Asp Ile Lys Ala Glu Ile Tyr Ser
    370                 375                 380

Phe Gly Ile Val Leu Trp Glu Ile Ala Thr Gly Lys Ile Pro Phe Glu
385                 390                 395                 400

Gly Cys Asp Ser Lys Lys Ile Arg Glu Leu Val Ala Glu Asp Lys Lys
                405                 410                 415

Gln Glu Pro Val Gly Gln Asp Cys Pro Glu Leu Leu Arg Glu Ile Ile
            420                 425                 430

Asn Glu Cys Arg Ala His Glu Pro Ser Gln Arg Pro Ser Val Asp Gly
        435                 440                 445

Ile Leu Glu Arg Leu Ser Ala Val Glu Glu Ser Thr Asp Lys Lys Val
    450                 455                 460

<210>  3
<211>  471
<212>  PRT
<213>  Homo sapiens

<400>  3

<210>  4
<211>  263
<212>  PRT
<213>  Homo sapiens

<400>  4

Met Glu Asn Leu Lys His Ile Ile Thr Leu Gly Gln Val Ile His Lys
1               5                   10                  15

Arg Cys Glu Glu Met Lys Tyr Cys Lys Lys Gln Cys Arg Arg Leu Gly
            20                  25                  30

His Arg Val Leu Gly Leu Ile Lys Pro Leu Glu Met Leu Gln Asp Gln
        35                  40                  45

Gly Lys Arg Ser Val Pro Ser Glu Lys Leu Thr Thr Ala Met Asn Arg
    50                  55                  60

Phe Lys Ala Ala Leu Glu Glu Ala Asn Gly Glu Ile Glu Lys Phe Ser
65                  70                  75                  80

Asn Arg Ser Asn Ile Cys Arg Phe Leu Thr Ala Ser Gln Asp Lys Ile
                85                  90                  95

Leu Phe Lys Asp Val Asn Arg Lys Leu Ser Asp Val Trp Lys Glu Leu
            100                 105                 110

Ser Leu Leu Leu Gln Val Glu Gln Arg Met Pro Val Ser Pro Ile Ser
        115                 120                 125

Gln Gly Ala Ser Trp Ala Gln Glu Asp Gln Gln Asp Ala Asp Glu Asp
    130                 135                 140

Arg Arg Ala Phe Gln Met Leu Arg Arg Asp Asn Glu Lys Ile Glu Ala
145                 150                 155                 160

Ser Leu Arg Arg Leu Glu Ile Asn Met Lys Glu Ile Lys Glu Thr Leu
                165                 170                 175

Arg Gln Ser Leu Glu Ser Ser Ser Gly Lys Ser Pro Leu Glu Ile Ser

180                 185                 190

Arg Phe Lys Val Lys Asn Val Lys Thr Gly Ser Ala Ser Gly Cys Asn
        195                 200                 205

Ser Glu Lys Ile Arg Lys Leu Val Ala Val Lys Arg Gln Gln Glu Pro
    210                 215                 220

Leu Gly Glu Asp Cys Pro Ser Glu Leu Arg Glu Ile Ile Asp Glu Cys
225                 230                 235                 240

Arg Ala His Asp Pro Ser Val Arg Pro Ser Val Asp Glu Ile Leu Lys
                245                 250                 255

Lys Leu Ser Thr Phe Ser Lys
            260

SEQUENCE LISTING

<110>  Eberhard Karls Universität Tübingen

<120>  MODULATION OF MIXED LINEAGE KINASE DOMAIN-LIKE PROTEIN SIGNALING

<130>  U30959EP

<140>  EP20173672.5
<141>  2020-05-08

<160>  8

<170>  PatentIn version 3.5

<210>  1
<211>  472
<212>  PRT
<213>  mus musculus

<400>  1

Met Asp Lys Leu Gly Gln Ile Ile Lys Leu Gly Gln Leu Ile Tyr Glu
1               5                   10                  15

Gln Cys Glu Lys Met Lys Tyr Cys Arg Lys Gln Cys Gln Arg Leu Gly
            20                  25                  30

Asn Arg Val His Gly Leu Leu Gln Pro Leu Gln Arg Leu Gln Ala Gln
        35                  40                  45

Gly Lys Lys Asn Leu Pro Asp Asp Ile Thr Ala Ala Leu Gly Arg Phe
    50                  55                  60

Asp Glu Val Leu Lys Glu Ala Asn Gln Gln Ile Glu Lys Phe Ser Lys
65                  70                  75                  80

Lys Ser His Ile Trp Lys Phe Val Ser Val Gly Asn Asp Lys Ile Leu
                85                  90                  95

Phe His Glu Val Asn Glu Lys Leu Arg Asp Val Trp Glu Glu Leu Leu
            100                 105                 110

Leu Leu Leu Gln Val Tyr His Trp Asn Thr Val Ser Asp Val Ser Gln
        115                 120                 125

Pro Ala Ser Trp Gln Gln Glu Asp Arg Gln Asp Ala Glu Glu Asp Gly
    130                 135                 140

Asn Glu Asn Met Lys Val Ile Leu Met Gln Leu Gln Ile Ser Val Glu
145                 150                 155                 160

Glu Ile Asn Lys Thr Leu Lys Gln Cys Ser Leu Lys Pro Thr Gln Glu

                165                 170                 175

Ile Pro Gln Asp Leu Gln Ile Lys Glu Ile Pro Lys Glu His Leu Gly
            180                 185                 190

Pro Pro Trp Thr Lys Leu Lys Thr Ser Lys Met Ser Thr Ile Tyr Arg
        195                 200                 205

Gly Glu Tyr His Arg Ser Pro Val Thr Ile Lys Val Phe Asn Asn Pro
    210                 215                 220

Gln Ala Glu Ser Val Gly Ile Val Arg Phe Thr Phe Asn Asp Glu Ile
225                 230                 235                 240

Lys Thr Met Lys Lys Phe Asp Ser Pro Asn Ile Leu Arg Ile Phe Gly
                245                 250                 255

Ile Cys Ile Asp Gln Thr Val Lys Pro Pro Glu Phe Ser Ile Val Met
            260                 265                 270

Glu Tyr Cys Glu Leu Gly Thr Leu Arg Glu Leu Leu Asp Arg Glu Lys
        275                 280                 285

Asp Leu Thr Met Ser Val Arg Ser Leu Leu Val Leu Arg Ala Ala Arg
    290                 295                 300

Gly Leu Tyr Arg Leu His His Ser Glu Thr Leu His Arg Asn Ile Ser
305                 310                 315                 320

Ser Ser Ser Phe Leu Val Ala Gly Gly Tyr Gln Val Lys Leu Ala Gly
                325                 330                 335

Phe Glu Leu Ser Lys Thr Gln Asn Ser Ile Ser Arg Thr Ala Lys Ser
            340                 345                 350

Thr Lys Ala Glu Arg Ser Ser Ser Thr Ile Tyr Val Ser Pro Glu Arg
        355                 360                 365

Leu Lys Asn Pro Phe Cys Leu Tyr Asp Ile Lys Ala Glu Ile Tyr Ser
    370                 375                 380

Phe Gly Ile Val Leu Trp Glu Ile Ala Thr Gly Lys Ile Pro Phe Glu
385                 390                 395                 400

Gly Cys Asp Ser Lys Lys Ile Arg Glu Leu Val Ala Glu Asp Lys Lys
                405                 410                 415

Gln Glu Pro Val Gly Gln Asp Cys Pro Glu Leu Leu Arg Glu Ile Ile
            420                 425                 430

Asn Glu Cys Arg Ala His Glu Pro Ser Gln Arg Pro Ser Val Asp Gly
        435                 440                 445

Arg Ser Leu Ser Gly Arg Glu Arg Ile Leu Glu Arg Leu Ser Ala Val
    450                 455                 460

Glu Glu Ser Thr Asp Lys Lys Val
465                 470

<210>  2
<211>  464
<212>  PRT
<213>  Mus musculus

<400>  2

Met Asp Lys Leu Gly Gln Ile Ile Lys Leu Gly Gln Leu Ile Tyr Glu
1               5                   10                  15

Gln Cys Glu Lys Met Lys Tyr Cys Arg Lys Gln Cys Gln Arg Leu Gly
            20                  25                  30

Asn Arg Val His Gly Leu Leu Gln Pro Leu Gln Arg Leu Gln Ala Gln
        35                  40                  45

Gly Lys Lys Asn Leu Pro Asp Asp Ile Thr Ala Ala Leu Gly Arg Phe
    50                  55                  60

Asp Glu Val Leu Lys Glu Ala Asn Gln Gln Ile Glu Lys Phe Ser Lys
65                  70                  75                  80

Lys Ser His Ile Trp Lys Phe Val Ser Val Gly Asn Asp Lys Ile Leu
                85                  90                  95

Phe His Glu Val Asn Glu Lys Leu Arg Asp Val Trp Glu Glu Leu Leu
            100                 105                 110

Leu Leu Leu Gln Val Tyr His Trp Asn Thr Val Ser Asp Val Ser Gln
        115                 120                 125

Pro Ala Ser Trp Gln Gln Glu Asp Arg Gln Asp Ala Glu Glu Asp Gly
    130                 135                 140

Asn Glu Asn Met Lys Val Ile Leu Met Gln Leu Gln Ile Ser Val Glu
145                 150                 155                 160

Glu Ile Asn Lys Thr Leu Lys Gln Cys Ser Leu Lys Pro Thr Gln Glu
                165                 170                 175

Ile Pro Gln Asp Leu Gln Ile Lys Glu Ile Pro Lys Glu His Leu Gly
            180                 185                 190

Pro Pro Trp Thr Lys Leu Lys Thr Ser Lys Met Ser Thr Ile Tyr Arg
        195                 200                 205

Gly Glu Tyr His Arg Ser Pro Val Thr Ile Lys Val Phe Asn Asn Pro
    210                 215                 220

Gln Ala Glu Ser Val Gly Ile Val Arg Phe Thr Phe Asn Asp Glu Ile
225                 230                 235                 240

Lys Thr Met Lys Lys Phe Asp Ser Pro Asn Ile Leu Arg Ile Phe Gly
                245                 250                 255

Ile Cys Ile Asp Gln Thr Val Lys Pro Pro Glu Phe Ser Ile Val Met
            260                 265                 270

Glu Tyr Cys Glu Leu Gly Thr Leu Arg Glu Leu Leu Asp Arg Glu Lys
        275                 280                 285

Asp Leu Thr Met Ser Val Arg Ser Leu Leu Val Leu Arg Ala Ala Arg
    290                 295                 300

Gly Leu Tyr Arg Leu His His Ser Glu Thr Leu His Arg Asn Ile Ser
305                 310                 315                 320

Ser Ser Ser Phe Leu Val Ala Gly Gly Tyr Gln Val Lys Leu Ala Gly
                325                 330                 335

Phe Glu Leu Ser Lys Thr Gln Asn Ser Ile Ser Arg Thr Ala Lys Ser
            340                 345                 350

Thr Lys Ala Glu Arg Ser Ser Ser Thr Ile Tyr Val Ser Pro Glu Arg
        355                 360                 365

Leu Lys Asn Pro Phe Cys Leu Tyr Asp Ile Lys Ala Glu Ile Tyr Ser
    370                 375                 380

Phe Gly Ile Val Leu Trp Glu Ile Ala Thr Gly Lys Ile Pro Phe Glu
385                 390                 395                 400

Gly Cys Asp Ser Lys Lys Ile Arg Glu Leu Val Ala Glu Asp Lys Lys
                405                 410                 415

Gln Glu Pro Val Gly Gln Asp Cys Pro Glu Leu Leu Arg Glu Ile Ile
            420                 425                 430

Asn Glu Cys Arg Ala His Glu Pro Ser Gln Arg Pro Ser Val Asp Gly
        435                 440                 445

Ile Leu Glu Arg Leu Ser Ala Val Glu Glu Ser Thr Asp Lys Lys Val
    450                 455                 460

<210> 3
<211> 471
<212> PRT
<213> Homo sapiens

<400> 3

<210>  4
<211>  263
<212>  PRT
<213>  Homo sapiens

<400>  4

Met Glu Asn Leu Lys His Ile Ile Thr Leu Gly Gln Val Ile His Lys
1               5                   10                  15

Arg Cys Glu Glu Met Lys Tyr Cys Lys Lys Gln Cys Arg Arg Leu Gly
            20                  25                  30

His Arg Val Leu Gly Leu Ile Lys Pro Leu Glu Met Leu Gln Asp Gln
        35                  40                  45

Gly Lys Arg Ser Val Pro Ser Glu Lys Leu Thr Thr Ala Met Asn Arg
    50                  55                  60

Phe Lys Ala Ala Leu Glu Glu Ala Asn Gly Glu Ile Glu Lys Phe Ser
65                  70                  75                  80

Asn Arg Ser Asn Ile Cys Arg Phe Leu Thr Ala Ser Gln Asp Lys Ile
                85                  90                  95

Leu Phe Lys Asp Val Asn Arg Lys Leu Ser Asp Val Trp Lys Glu Leu
            100                 105                 110

Ser Leu Leu Leu Gln Val Glu Gln Arg Met Pro Val Ser Pro Ile Ser
        115                 120                 125

Gln Gly Ala Ser Trp Ala Gln Glu Asp Gln Gln Asp Ala Asp Glu Asp
    130                 135                 140

Arg Arg Ala Phe Gln Met Leu Arg Arg Asp Asn Glu Lys Ile Glu Ala
145                 150                 155                 160

Ser Leu Arg Arg Leu Glu Ile Asn Met Lys Glu Ile Lys Glu Thr Leu
                165                 170                 175

Arg Gln Ser Leu Glu Ser Ser Ser Gly Lys Ser Pro Leu Glu Ile Ser
                180                 185                 190

Arg Phe Lys Val Lys Asn Val Lys Thr Gly Ser Ala Ser Gly Cys Asn
            195                 200                 205

Ser Glu Lys Ile Arg Lys Leu Val Ala Val Lys Arg Gln Gln Glu Pro
    210                 215                 220

Leu Gly Glu Asp Cys Pro Ser Glu Leu Arg Glu Ile Ile Asp Glu Cys
225                 230                 235                 240

Arg Ala His Asp Pro Ser Val Arg Pro Ser Val Asp Glu Ile Leu Lys
                245                 250                 255

Lys Leu Ser Thr Phe Ser Lys
            260

<210>  5
<211>  8
<212>  PRT
<213>  mus musculus

<400>  5

Arg Ser Leu Ser Gly Arg Glu Arg
1               5

<210>  6
<211>  8
<212>  PRT
<213>  mus musculus

<400>  6

Met Ala Phe Glu Glu Arg Gln Lys
1               5

<210>  7
<211>  8
<212>  PRT
<213>  mus musculus

<400>  7

Arg Ser Leu Ser Gly Arg Glu Arg
1               5

<210>  8
<211>  8
<212>  PRT
<213>  Homo sapiens

<400>  8

Arg Ser Leu Ser Gly Arg Glu Arg
1               5

## Description

### FIELD OF THE INVENTION

The invention is based on a method of modulating the activation or inhibition of Mixed lineage kinase domain-like (MLKL) protein, or a MLKL variant protein, via modulating the intramolecular interaction between the C-terminal helix (Hc) of the psK domain and a hydrophobic groove in the MLKL protein. The invention provides methods and compounds to selectively target the herein firstly disclosed intramolecular interaction of MLKL protein. Based on the herein disclosed essential intramolecular rearrangement of MLKL, the invention provides small molecules capable of specifically inhibiting mouse or human MLKL. The invention provides uses, including research and medical applications such as treatments, of MLKL driven conditions including necroptosis, cell trafficking, pathological immune responses and/or inflammation.

### DESCRIPTION

Cell death is fundamental for development, tissue homeostasis and immunity in multicellular organisms. Necroptosis is a form of regulated cell death that results in release of the inflammatory cellular contents after plasma membrane permeabilization, thereby triggering immune responses (1, 2). The medical importance of necroptosis has recently been illustrated by its connection to cancer (3, 4), inflammation (5), neurodegenerative diseases (6, 7) and pathogen infections (8, 9). Because of this, targeting necroptosis signaling is of great scientific and therapeutic interest and elucidating how it is regulated at the molecular level will help designing novel therapies for human health.

Necroptosis can be triggered via different receptors, including the toll-like receptors 3/4 (TLR3/4) and death receptors such as tumor necrosis factor receptor 1 (TNFRi), TRAIL receptors 1 and 2 (TRAIL-R1/2), and CD95 (Fas/APO-1) (10), and via the DNA-dependent activator of interferon-regulatory factors (DAI), under conditions of compromised caspase 8 activity. All these pathways converge in the formation of the necrosome, which contains active, phosphorylated Receptor-Interacting Protein kinase 3 (RIP3) and Mixed Lineage Kinase domain-Like (MLKL) (11-14). MLKL is essential for necroptosis and it is the most downstream effector of this form of cell death identified to date. RIP3-mediated phosphorylation activates MLKL and drives its oligomerization and translocation from the cytosol to the plasma membrane. Although activation of MLKL is essential for the key step of plasma membrane permeabilization and the execution of cell death (12, 15, 16), its mechanism of action remains unclear. Competing models propose that MLKL either indirectly (17, 18) or directly (19, 20) induces plasma membrane permeabilization to mediate cell death.

MLKL is a member of the pseudokinases family, a group of proteins that are catalytically deficient variants of kinases but keep the ability to bind ATP to exert signal transduction and scaffolding functions (21, 22). Solving the structure of MLKL by X-ray crystallography revealed an N-terminal four helix bundle domain (4HB), a C-terminal pseudokinase domain (psK) containing the RIP3 phosphorylation site and a brace region that bridges them (21). The 4HB of MLKL acts as the killer domain, whereas the psK domain is critical to restrain the death-inducing capacity of MLKL in healthy conditions (23, 24). Mechanistically, it has been shown that phosphorylation at defined sites in the psK domain is required to induce brace opening, 4HB exposure and cell death (23). However, how the psK domain regulates MLKL activation and the nature of the conformational changes in the psK that are enabled by phosphorylation remain unknown.

The interest in MLKL inhibition for blocking necroptosis has recently escalated because, to date, MLKL is the only effector solely implicated in this cell death pathway (13, 23, 25). Whereas inhibiting the kinase activity of RIP3 has been shown to block necroptosis, doing so fails to prevent cell death because it removes a RIP3-kinase-dependent regulatory control on caspase 8 activation, leading to aberrant apoptosis (26). Blocking the activity of the upstream kinase RIP1 has shown to be a promising therapeutic strategy for several indications (27-29). However, this strategy may not come without adverse effects, as it not only inhibits necroptosis, but also RIP1-kinase-dependent gene activation and/or apoptosis associated with its role as master regulator of all outputs of receptor-mediated signaling (30, 31). It is possible that inhibiting these additional RIP1-kinase-dependent pathways may act synergistically in certain diseases with the inhibition of necroptosis, yet this would be accidental. Hence, inhibiting the most downstream, possibly unique, sole necroptosis effector molecule MLKL would constitute the most specific means of necroptosis inhibition.

Uncovering the structural determinants required for MLKL regulation is key to discover new drugs interfering exclusively with necroptotic cell death. Targeting the ATP-binding site of the psK domain (23) is currently used as a strategy to develop new MLKL modulators. However, this approach suffers from low specificity because this pocket shares structural similarities with other (pseudo)kinases, which may cause undesirable off-target effects. Therefore, there is a need for the identification of alternative druggable sites on the structure of MLKL.

Thus, it is an objective of the invention to provide novel means and methods to modulate MLKL activity in order to devise their application not only in the modulation of necroptotic cell death, but also in the treatment or management of conditions associated with cell trafficking, pathological immune responses and/or inflammation.

### BRIEF DESCRIPTION OF THE INVENTION

Generally, and by way of brief description, the main aspects of the present invention can be described as follows:

In **a first aspect,** the invention pertains to a method for modulating the activation of Mixed lineage kinase domain-like (MLKL) protein, or a MLKL variant protein, wherein the MLKL protein, or MLKL variant protein, comprises at least an N-terminal four helix bundle (4HB) domain, a C-terminal pseudo kinase (psK) domain, connected by a brace region, and wherein the method comprises the modulation of the intramolecular interaction between the C-terminal helix (Hc) of the psK domain and a hydrophobic groove, and wherein the intramolecular interaction involves MLKL amino acid residues located in the 4HB domain, the brace region and the psK domain.

In **a second aspect,** the invention pertains to a method for modulating the activation capacity of necroptosis in a cell, the method comprising the step of contacting the cell with a dominant negative MLKL protein, a MLKL modulating compound or MLKL modulating composition, wherein said MLKL modulating compound or MLKL modulating composition when contacted with the cell modulates an intramolecular interaction between the C-terminal helix (Hc) of the psK domain and a hydrophobic groove wherein the intramolecular interaction involves MLKL amino acid residues located in the 4HB domain, the brace region and the psK domain of an MLKL protein, or a MLKL variant protein, in the cell.

In **a third aspect,** the invention pertains to a method for identifying a compound capable of modulating activation of MLKL, the method comprising
- Bringing into contact an MLKL protein, or an MLKL variant protein, and/or a cell expressing an MLKL protein, or an MLKL variant protein, with a candidate compound, wherein the MLKL protein, or the MLKL variant protein, comprises at least an N-terminal four helix bundle (4HB) domain, a C-terminal pseudo kinase (psK) domain, connected by a brace region;
- Optionally, activating the MLKL protein, or the MLKL variant protein;
- Determining the intramolecular interaction between the C-terminal helix (Hc) of the psK domain and a hydrophobic groove of the MLKL protein, or the MLKL variant protein, when contacted with the candidate compound;
wherein: (i) the an altered intramolecular interaction between Hc and the hydrophobic groove of the MLKL protein, or the MLKL variant protein, contacted with the candidate compound compared to an MLKL protein, or the MLKL variant protein, not contacted with the candidate compound, and/or (ii) an altered intramolecular interaction between Hc and the hydrophobic groove of the MLKL protein, or the MLKL variant protein, expressed by a cell contacted with the candidate compound compared to a cell not contacted with the candidate compound; indicated that the candidate compound is a compound capable of modulating the activation of MLKL.

In **a fourth aspect,** the invention pertains to a method for identifying a compound capable of modulating activation of MLKL, the method comprising:
- Bringing into contact a MLKL Hc domain with a candidate compound,
- Determining a specific binding of the candidate compound to the MLKL Hc domain, wherein a specific binding of the candidate compound to the MLKL Hc domain compared to the binding to an unrelated protein domain indicates that the candidate compound is capable of modulating activation of MLKL.

In **a fifth aspect,** the invention pertains to a method for identifying a compound capable of modulating activation of MLKL, the method comprising:
- Bringing into contact a candidate compound with a MLKL hydrophobic groove domain which is a protein domain comprising amino acid residues from the 4HB domain, the brace region and the psK domain,
- Determining a specific binding of the candidate compound to the MLKL hydrophobic groove domain,
wherein a specific binding of the candidate compound to the MLKL hydrophobic groove domain compared to the binding to an unrelated protein domain indicates that the candidate compound is capable of modulating activation of MLKL.

In **a sixth aspect,** the invention pertains to a method for treating a disease associated with necroptosis, inflammation, pathological immune response and/or trafficking in a subject, the method comprising performing in the subject a method according to any one of the preceding aspects, wherein the cell is a cell associated with the disease. In an alternative embodiment of the sixth aspect, the invention pertains to substance/composition for use in treating a disease associated with necroptosis, inflammation, pathological immune response and/or trafficking in a subject, wherein the substance or composition has an activity as a modulator of MLKL as disclosed herein.

### DETAILED DESCRIPTION OF THE INVENTION

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a method for modulating the activation of Mixed lineage kinase domain-like (MLKL) protein, or a MLKL variant protein, wherein the MLKL protein, or MLKL variant protein, comprises at least an N-terminal four helix bundle (4HB) domain, a C-terminal pseudo kinase (psK) domain, connected by a brace region, and wherein the method comprises the modulation of the intramolecular interaction between the C-terminal helix (Hc) of the psK domain and a hydrophobic groove, and wherein the intramolecular interaction involves MLKL amino acid residues located in the 4HB domain, the brace region and the psK domain.

The method in some preferred aspects and embodiments is a non-therapeutic method. In addition, the term "non-therapeutic" in the present invention refers to a concept that does not include medical practice medical treatment of a human body or an animal body through therapy, such as the method for research purposes or for screening approaches.

The present invention is based on the surprising find that two murine isoforms of MLKL that molecularly only differ in the presence of an eight amino acid stretch at the C-terminal helix Hc are different in their function. When a known necroptosis trigger is applied, the MLKL version containing the insertion remains inactive, whereas the one devoid of this sequence is activated. Analyzing in detail the underlying molecular mechanism resulted in the discovery of a previously unrecognized interaction of Hc with a hydrophobic groove in MLKL that is essential for its capacity to induce necroptosis - this leads to the inventive aspects and embodiments of the present disclosure. This new insight further informed the design of a novel class of potent and species-specific inhibitors for both, murine and human MLKL, which are shown to substantially ameliorate necroptosis-driven dermatitis. The invention therefore provides a novel and highly specific strategy to prevent necroptosis therapeutically. The invention in certain of its aspects and embodiments is for use in medicine and in research.

In an alternative first aspect of the invention, there is provided a substance or composition for use in the method of the above first aspect, wherein said substance or composition is a modulator of said intramolecular interaction between the C-terminal helix (Hc) of the psK domain and a hydrophobic groove, and wherein the intramolecular interaction involves MLKL amino acid residues located in the 4HB domain, the brace region and the psK domain. Such use is preferably a use in medicine, for example, a use in the treatment and/or prevention of a disorder associated with MLKL activation (such as necroptosis).

In context of the present invention the term Mixed lineage kinase domain-like (MLKL) protein, shall refer to a protein as shown in SEQ ID NO: 1 or 2 (mouse MLKL isoforms 1 and 2) or SEQ ID NO: 3 or 4 (human MLKL isoforms 1 and 2). The protein identities can also be derived from the UniProt database in the version of February 2020 (www.uniprot.org). For the mouse MLKL isoform 1 under the accession number Q9D2Y4-1, for mouse MLKL isoform 2 under the accession number Q9D2Y4-2; for the human MLKL isoform 1 under the accession number Q8NB16-1, and for the human MLKL isoform 2 under the accession number Q8NB16-2.

In some preferred aspects and embodiments of the invention the MLKL protein, or a variant protein thereof, is a human MLKL protein.

The term "a MLKL variant protein", or "variant MLKL protein" or similar expression, shall refer to any MLKL protein homologs, paralogs or orthologues of a human MLKL. In preferred embodiments a MLKL variant protein is a protein having an amino acid sequence with at least 50, 60, 70, 80, 85, 90, 95, 96, 97, 98, or 99 percent sequence identity to a sequence shown in one of SEQ ID NO: 1 to 4.

The term "modulating activation of MLKL" pertains to a change of activity of a MLKL protein selected from an activity as a necroptosis regulator, and without being bound to a specific theory, is being modulated by the modulation of a three-dimensional intramolecular conformation of the MLKL protein. The term "modulating" in this context preferably pertains to an "inhibition" of one or more activities of the MLKL protein, such as an inhibition of the necroptosis mediating function of the MLKL protein. Moreover, and in alternative or additional embodiments, the activity can be seen in the permeabilization of a cell plasma membrane induced by the MLKL protein. Furthermore, in additional or alternative embodiments, the activity of the MLKL protein of the invention is an interaction of the C-terminal helix (Hc) located in the pseudo kinase domain (psK) of the MLKL protein with the 4HB domain, preferably wherein the interaction of the Hc has a necroptosis agonistic activity. More specifically, the modulating includes a modulation of the intramolecular interaction of the Hc with a hydrophobic groove in the MLKL protein wherein the intramolecular interaction involves MLKL amino acid residues located in the 4HB domain, the brace region and the psK domain. Hence, in preferred embodiments of the invention the modulation of activation is a decrease in activation, or a decrease in the ability to be activated, and wherein the modulation of the intramolecular interaction is a reduced or impaired intramolecular interaction.

In yet preferred embodiments of the invention the modulation of the intramolecular interaction may involve any of the following procedures:
- Introduction of one or more amino acid exchanges into the Hc and/or the hydrophobic groove of the MLKL protein, or MLKL variant protein; and/or
- Contacting the MLKL protein, or MLKL variant protein, with a compound known to bind to MLKL at least in part at the Hc and/or the hydrophobic groove and thereby modulate their intramolecular interaction.

In the context of the present invention the term "hydrophobic groove" or "MLKL hydrophobic groove" pertains to a previously not defined structural motif of MLKL proteins, specifically of mouse or human MLKL proteins. Specifically, the hydrophobic groove comprises amino acid residues from the 4HB domain, the brace region and the psK domain, of the MLKL protein, and in the event the MLKL protein, or MLKL variant protein, is human MLKL1 (SEQ ID NO: 3), such amino acids are located between amino acid 80 and 100, more preferably 80 to 90, and most preferably are amino acids 81, 82, 83, 85, 86, 87, 89 or 90 from the 4HB; such amino acids are located between amino acid 120 and 190, more preferably 140 to 160, and most preferably are amino acids 148, 152, 155 or 156 from the brace region; such amino acids are located between amino acid 190 and 471, more preferably 300 to 450, and most preferably are amino acids 303, 307, 310, 311, 314, 315, 317, 318, 321, 322, 391, 392, 394, 395, 398, 402, 438, 440, 441, 443, 444, 445, 447 from the psK domain. Most importantly it is understood that the hydrophobic groove involves at least one amino acid of the aforementioned domain regions in the human MLKL protein. In the event the MLKL protein, or MLKL variant protein, is mouse MLKL2 (SEQ ID NO: 2), such amino acids are located between amino acid 20 and 100, more preferably 20 to 90, and most preferably are amino acids 23, 79, 80, 81, 84, 85, 86, 87, 88 or 89 from the 4HB; such amino acids are located between amino acid 120 and 190, more preferably 140 to 160, and most preferably are amino acids 147, 150, 151, 154 or 155 from the brace region; such amino acids are located between amino acid 190 and 464, more preferably 290 to 440, and most preferably are amino acids 296, 299, 300, 303, 304, 306, 307, 310, 311, 378, 382, 385, 389, 427, 428, 430, 431, 434 or 435 from the psK domain. Most preferably the present invention pertains to the human MLKL protein. It is understood that both for human and mouse MLKL proteins, the preferred embodiments include a combination of the most preferred amino acid positions of the 4HB, the brace region and the psK.

In some embodiments of the invention the Hc is located at the C-terminal end of the psK domain, and in the event the MLKL protein, or MLKL variant protein, is human MLKL1 (SEQ ID NO: 3), is between amino acids 400 to 471, preferably 450 to 471, most preferably between 460 to 471, and most preferably is between amino acid 458 to 468; and in the event the MLKL protein, or MLKL variant protein, is mouse MLKL2 (SEQ ID NO: 2), is between amino acids 400 to 464, preferably 420 to 464, most preferably between 430 to 460, and most preferably is between amino acids 445 to 455.

In preferred embodiments of the invention the modulating the intramolecular interaction comprises contacting the MLKL protein, or MLKL variant protein, with a compound selected from formula I or II, as well as derivatives, and solvates, salts, stereoisomers, complexes, polymorphs, crystalline forms, racemic mixtures, diastereomers, enantiomers, tautomers, isotopically labelled forms, prodrugs, and combinations thereof:

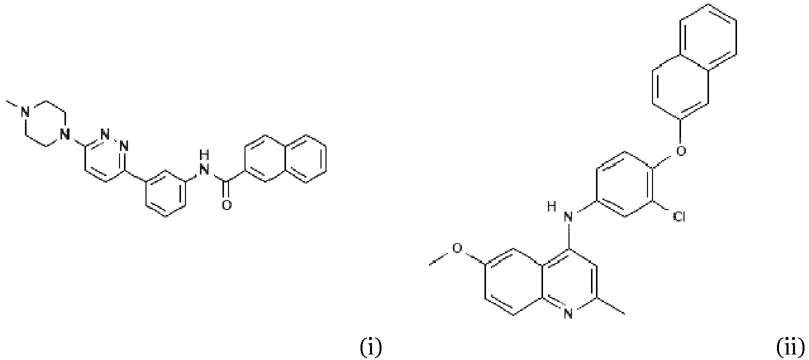

Preferably the above compound (I) [*N*-[3-[6-(4-methylpiperazin-1-yl)pyridazin-3-yl]phenyl]naphthalene-2-carboxamide] is an inhibitor of human MLKL protein, and the above compound (ii) [*N*-(3-chloro-4-naphthalen-2-yloxyphenyl)-6-methoxy-2-methylquinolin-4-amine] is an inhibitor of mouse MLKL protein.

In preferred embodiments of the invention said modulation of intramolecular interaction does not involve a modulation of interaction at the ATP-binding site or at the phosphorylation site within the MLKL protein, or MLKL variant protein; optionally, wherein the method additionally comprises an allosteric modulation of interaction at the ATP-binding site or at the phosphorylation site within the MLKL protein, or MLKL variant protein.

In **a second aspect,** the invention pertains to a method for modulating the activation capacity of necroptosis in a cell, the method comprising the step of contacting the cell with a MLKL modulating compound or MLKL modulating composition, wherein said MLKL modulating compound or MLKL modulating composition when contacted with the cell modulates an intramolecular interaction between the C-terminal helix (Hc) of the psK domain and a hydrophobic groove, wherein the hydrophobic groove is a 3 dimensional structural motif in the MLKL protein, or in the variant MLKL protein, that comprises and/or involves at least one amino acid residue from each of the 4HB domain, the brace region and the psK domain.

In preferred embodiments of the invention the impairment of the intramolecular interaction between the Hc and the hydrophobic groove of the MLKL protein, or the MLKL variant protein, in the cell impairs the activation capacity of necroptosis in the cell, for example impairs the permeabilization of the plasma membrane of the cell.

In another embodiment of the invention, the MLKL modulating compound or substance or MLKL modulating composition is selected from a polypeptide, peptide, glycoprotein, a peptidomimetic, an antigen binding construct (for example, an antibody, antibody-like molecule or other antigen binding derivative, or an or antigen binding fragment thereof), a nucleic acid such as a DNA or RNA, for example an antisense or inhibitory DNA or RNA, a ribozyme, an RNA or DNA aptamer, RNAi, siRNA, shRNA and the like, including variants or derivatives thereof such as a peptide nucleic acid (PNA), a genetic construct for targeted gene editing, such as a CRISPR/Cas9 construct and/or a guide nucleic acid (gRNA or gDNA) and/or tracrRNA, or a small molecular compound, preferably a small molecule having a molecular weight of less than 10 kD, more preferably of less than 2kD, most preferably of less than 1kD, or any combination of the above.

Preferably, the MLKL modulating compound or MLKL modulating composition is a compound/substance or composition of multiple compounds for the targeted introduction of mutations into the gene encoding the MLKL protein, or the MLKL variant protein, in the cell. For example, the targeted introduction of mutations comprises the targeted introduction of mutations into the sequence encoding the Hc and/or hydrophobic groove.

In preferred embodiments of this aspect, the Hc is located at the C-terminal end of the psK domain, and in the event the MLKL protein, or MLKL variant protein, is human MLKL1 (SEQ ID NO: 3), is between amino acids 400 to 471, preferably 450 to 471, most preferably between 460 to 471, most preferably is between amino acid 458 to 468; and in the event the MLKL protein, or MLKL variant protein, is mouse MLKL2 (SEQ ID NO: 2), is between amino acids 400 to 464, preferably 420 to 464, most preferably between 430 to 460, and most preferably is between amino acids 445 to 455.

In another preferred embodiment of this aspect, and the other aspects as well, the hydrophobic groove wherein the hydrophobic groove comprises at least one amino acid residue from each of the 4HB domain, the brace region and the psK domain, and
- in the event the MLKL protein, or MLKL variant protein, is human MLKL1 (SEQ ID NO: 3), the at least one amino acid residue of the 4HB domain is selected from a sequence between amino acid 80 and 100, more preferably 80 to 90, and most preferably are amino acids 81, 82, 83, 85, 86, 87, 89 or 90 of human MLKL; the at least one amino acid residue of the brace region is selected from a sequence between amino acid 120 and 190, more preferably 140 to 160, and most preferably are amino acids 148, 152, 155 or 156 from human MLKL; and the at least one amino acid residue of the psK domain is selected from a sequence between amino acid 190 and 471, more preferably 300 to 450, and most preferably are amino acids 303, 307, 310, 311, 314, 315, 317, 318, 321, 322, 391, 392, 394, 395, 398, 402, 438, 440, 441, 443, 444, 445, 447 from human MLKL; or
- in the event the MLKL protein, or MLKL variant protein, is mouse MLKL2 (SEQ ID NO: 2), the at least one amino acid residue of the 4HB domain is selected from a sequence between amino acid 20 and 100, more preferably 20 to 90, and most preferably are amino acids 23, 79, 80, 81, 84, 85, 86, 87, 88 or 89; the at least one amino acid residue of the brace region is selected from a sequence between amino acid 120 and 190, more preferably 140 to 160, and most preferably are amino acids 147, 150, 151, 154 or 155; the at least one amino acid residue of the psK domain is selected from a sequence between amino acid 190 and 464, more preferably 290 to 440, and most preferably are amino acids 296, 299, 300, 303, 304, 306, 307, 310, 311, 378, 382, 385, 389, 427, 428, 430, 431, 434 or 435.

In **a third aspect,** the invention pertains to a method for identifying a compound capable of modulating activation of MLKL, the method comprising
- Bringing into contact an MLKL protein, or an MLKL variant protein, and/or a cell expressing an MLKL protein, or an MLKL variant protein, with a candidate compound, wherein the MLKL protein, or the MLKL variant protein, comprises at least an N-terminal four helix bundle (4HB) domain, a C-terminal pseudo kinase (psK) domain, connected by a brace region;
- Optionally, activating the MLKL protein, or the MLKL variant protein;
- Determining the intramolecular interaction between the C-terminal helix (Hc) of the psK domain and a hydrophobic groove of the MLKL protein, or the MLKL variant protein, when contacted with the candidate compound;
wherein: (i) the an altered intramolecular interaction between Hc and the hydrophobic groove of the MLKL protein, or the MLKL variant protein, contacted with the candidate compound compared to an MLKL protein, or the MLKL variant protein, not contacted with the candidate compound, and/or (ii) an altered intramolecular interaction between Hc and the hydrophobic groove of the MLKL protein, or the MLKL variant protein, expressed by a cell contacted with the candidate compound compared to a cell not contacted with the candidate compound; indicated that the candidate compound is a compound capable of modulating the activation of MLKL.

In one preferred embodiment, the MLKL protein, or variant MLKL protein, is a constitutively active MLKL protein, for example a phosphorylated MLKL protein.

It may further be preferred that step (ii) of the above method of the third aspect is mandatory and comprises phosphorylation of the MLKL protein, or the MLKL variant protein; and/or comprises inducing necroptosis in the cell expressing the MLKL protein, or MLKL variant protein, or alternatively comprises trafficking, pathological immune responses and/or inflammation.

The method according to any one of claims 15 to 17, wherein the candidate compound is selected from a polypeptide, peptide, glycoprotein, a peptidomimetic, an antigen binding construct (for example, an antibody, antibody-like molecule or other antigen binding derivative, or an or antigen binding fragment thereof), a nucleic acid such as a DNA or RNA, for example an antisense or inhibitory DNA or RNA, a ribozyme, an RNA or DNA aptamer, RNAi, siRNA, shRNA and the like, including variants or derivatives thereof such as a peptide nucleic acid (PNA), a genetic construct for targeted gene editing, such as a CRISPR/Cas9 construct and/or a guide nucleic acid (gRNA or gDNA) and/or tracrRNA, or a small molecular compound, preferably a small molecule having a molecular weight of less than 5 kD, more preferably of less than 2kD, most preferably of less than 1kD, or any combination of the above.

In **a fourth aspect,** the invention pertains to a method for identifying a compound capable of modulating activation of MLKL, the method comprising:
- Bringing into contact a MLKL Hc domain with a candidate compound,
- Determining a specific binding of the candidate compound to the MLKL Hc domain, wherein a specific binding of the candidate compound to the MLKL Hc domain compared to the binding to an unrelated protein domain indicates that the candidate compound is capable of modulating activation of MLKL.

In **a fifth aspect,** the invention pertains to a method for identifying a compound capable of modulating activation of MLKL, the method comprising:
- Bringing into contact a candidate compound with a MLKL hydrophobic groove domain which is a protein domain comprising amino acid residues from the 4HB domain, the brace region and the psK domain,
- Determining a specific binding of the candidate compound to the MLKL hydrophobic groove domain,
wherein a specific binding of the candidate compound to the MLKL hydrophobic groove domain compared to the binding to an unrelated protein domain indicates that the candidate compound is capable of modulating activation of MLKL.

In one embodiment the MLKL hydrophobic groove domain and/or the MLKL Hc domain are provided as full-length MLKL protein, or are provided as test proteins comprising the MLKL hydrophobic groove domain and/or the MLKL Hc domain, but not comprising the full psK and/or 4HB domain, more preferably the test protein is a MLKL mutant protein, such as a constitutively active MLKL protein, or a constitutively active MLKL variant protein.

In **a sixth aspect,** the invention pertains to a method for treating a disease associated with necroptosis, inflammation, pathological immune response and/or trafficking in a subject, the method comprising performing in the subject a method according to any one of the preceding aspects, wherein the cell is a cell associated with the disease. In an alternative embodiment of the sixth aspects, the invention pertains to substance/composition for use in treating a disease associated with necroptosis, inflammation, pathological immune response and/or trafficking in a subject, wherein the substance or composition has an activity as a modulator of MLKL as disclosed herein.

The method of treating a disease in preferred embodiments comprises a step of administering to the subject a therapeutically effective amount of the MLKL modulating compound or MLKL modulating composition.

In the various aspects and embodiments, the present invention pertains to a use in research or medicine, specifically in the prevention and/or treatment of a disease. In this context it is preferred that the disease is a disease associated with necroptosis and is preferably is selected from the group consisting of diseases of the bones, joints, connective tissue and cartilage, muscular diseases, skin diseases, cardiovascular diseases, circulatory diseases, hematological and vascular diseases, diseases of the lung, diseases of the gastro-intestinal tract, diseases of the liver, diseases of the pancreas, metabolic diseases, diseases of the kidneys, viral and bacterial infections, severe intoxications, degenerative diseases associated with the Acquired Immune Deficiency Syndrome (AIDS), disorders associated with aging, inflammatory diseases, auto-immune diseases, dental disorders, ophthalmic diseases or disorders, diseases of the audition tracts, diseases associated with mitochondria, and cancer, such as a solid cancer or lymphoid cancer, and cancer metastasis.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

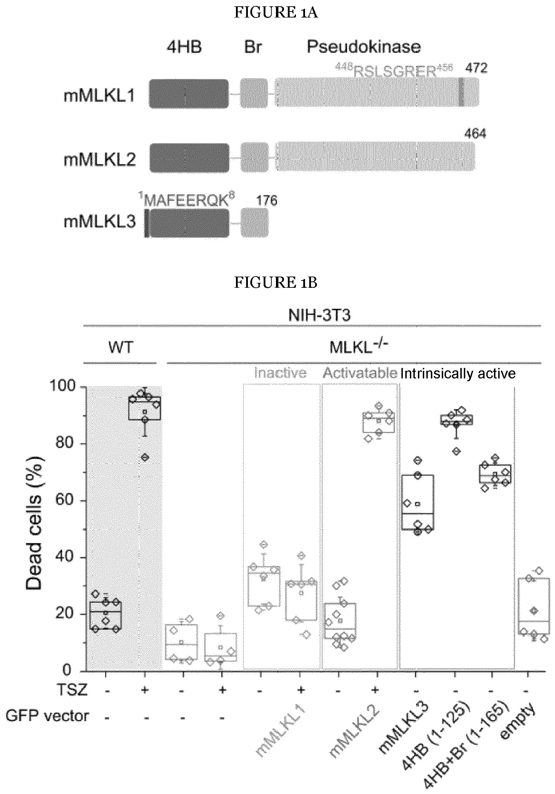

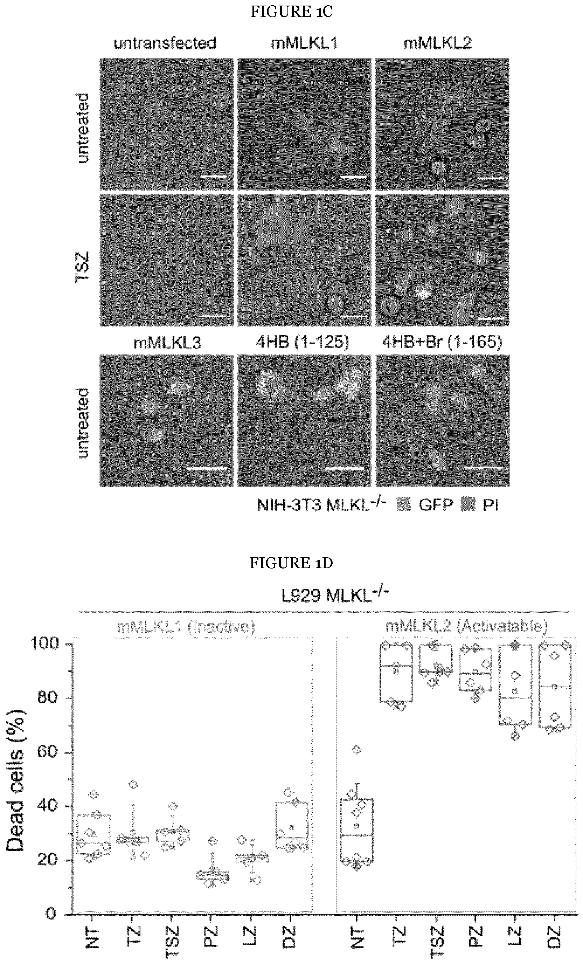

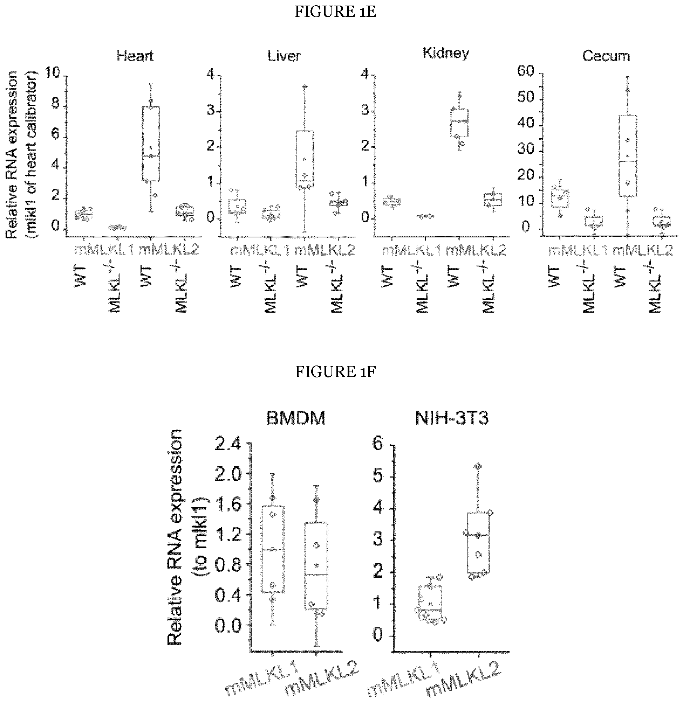

**Figure 1****:** shows that mouse MLKL isoforms have different necroptosis activity. A) Representation of mouse MLKL isoforms. B-C) Necroptosis response of NIH-3T3 MLKL ko cells transfected with one of the isoforms and treated with TSZ. B) Flow cytometry measurements. C) Confocal images. Pictures are representative of at least three independent experiments. Scale bar: 20 µm. D) Dead cell population of L929 MLKL ko cells transfected with one of the isoforms and treated with different necroptosis inducers. NT: non-treated, TZ (TNF activation: TNF+zVAD), TSZ (TNF activation: TNF+Smac+zVAD), PZ (TLR3 activation: dsRNA analogue poly(I:C)+zVAD), LZ (TLR4 activation: LPS+zVAD) and DZ (DAI activation: dsDNA analogue poly(A:T)+zVAD). MLKL ko cells were transfected with the different variants of mMLKL-GFP. The dead cell population was calculated from transfected cells. Individual values from at least three independent experiments are shown. E-F) Relative RNA levels of mMLKL1 compared with mMLKL2 in E) different organs from wild type (WT) or MLKL ko mice, F) BMDM and NIH-3T3 cells. Levels are relative to the average of mlkl1_RNA detected in the heart samples E) or in each cell line F). Primers to detect RNA for mMLKL1 were designed to specifically recognize the small sequence that is different to mMLKL2, located near the 3' end.

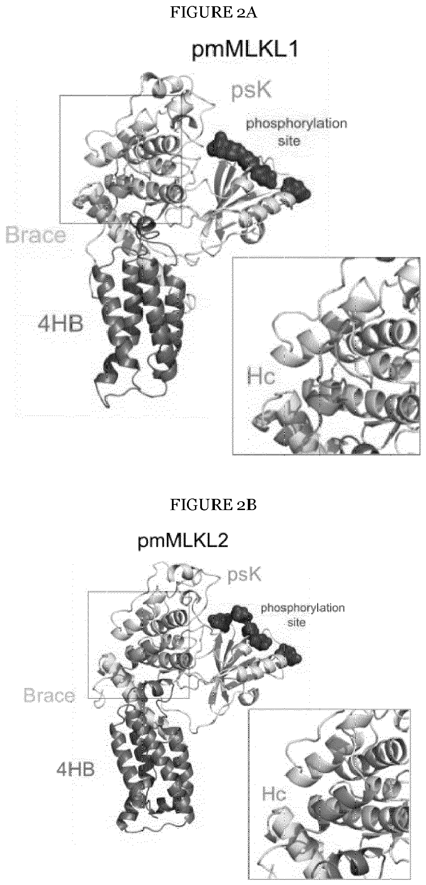

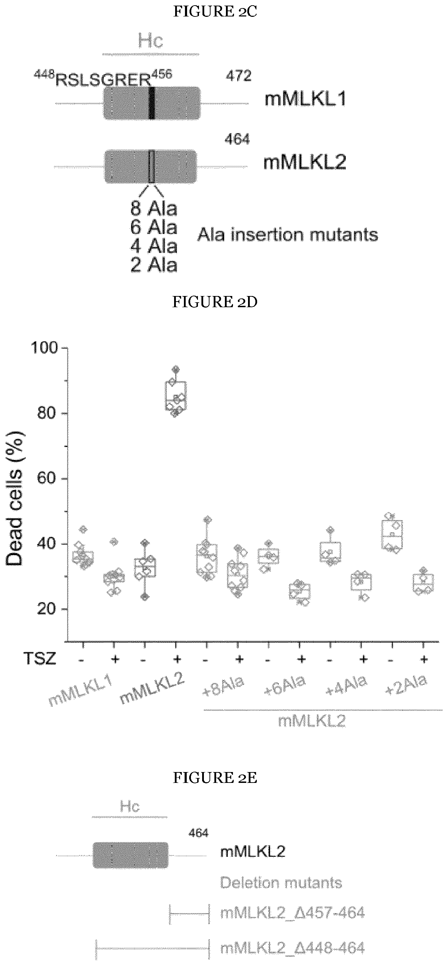

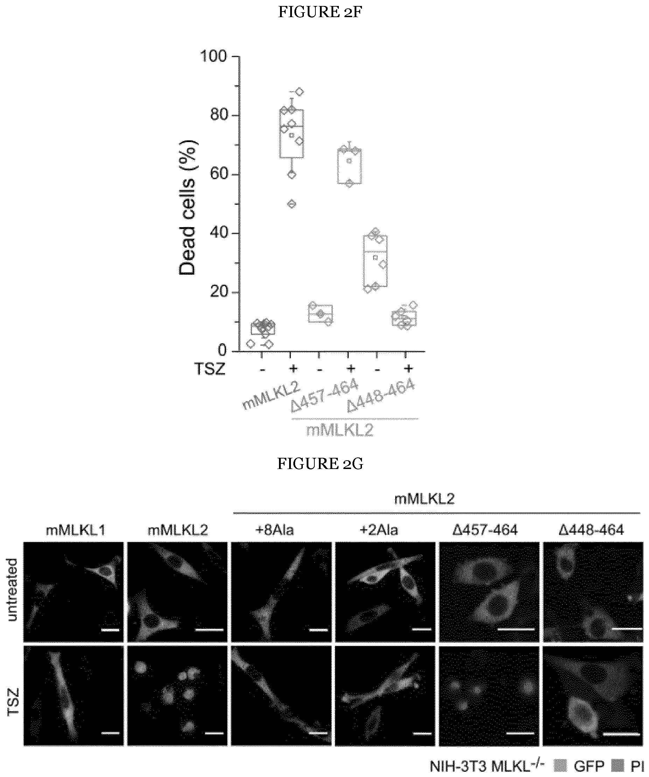

**Figure 2****:** shows that the Hc of mouse MLKL is essential for necroptosis. Model of A) phosphomimetic mMLKL1 (pmMLKL1) and B) phosphomimetic mMLKL2 (pmMLKL2). mMLKL2 structure was refined from the crystal structure (PDB: 4BTF) and mMLKL1 model was based on the refined structure of mMLKL2. Phosphomimetic mutations are represented in red: S228E, S345D, S347D, T349D, and S352D. C) Schematic representation of the Ala insertion mutants. D) Effect of Ala insertion in the Hc of mMLKL2 on necroptotic cell death. E) Schematic representation of C-terminal deletion mutants. F) Effect of deletion of different C-terminal segments of mMLKL2 on necroptotic cell death. NIH-3T3 MLKL ko cells were transfected with the different variants of mMLKL-GFP and TSZ-treated. The dead cell population was calculated from transfected cells. Individual values from at least three independent experiments are shown. G) Images from confocal microscopy. Pictures are representative of at least three independent experiments. Scale bar: 20 µm.

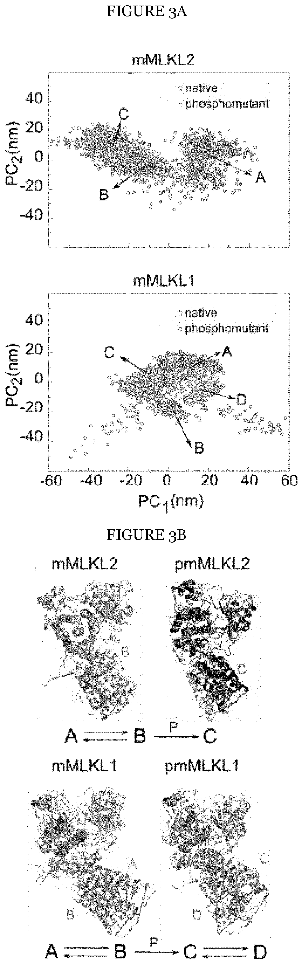

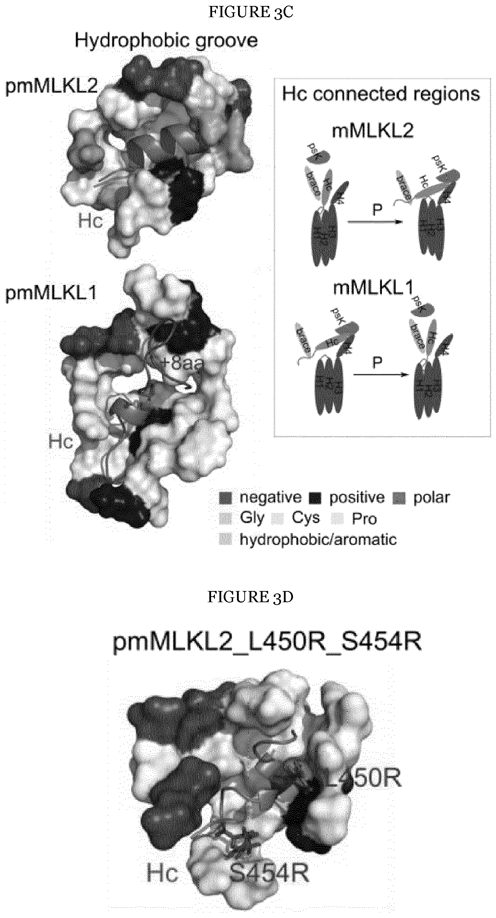

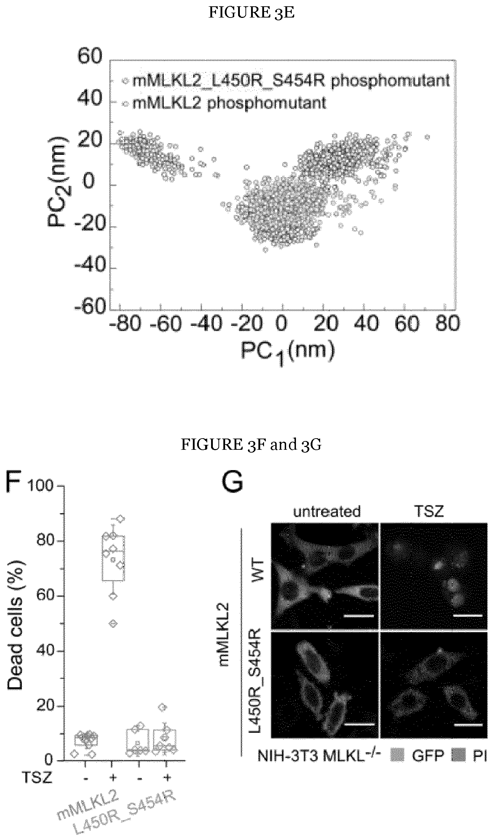

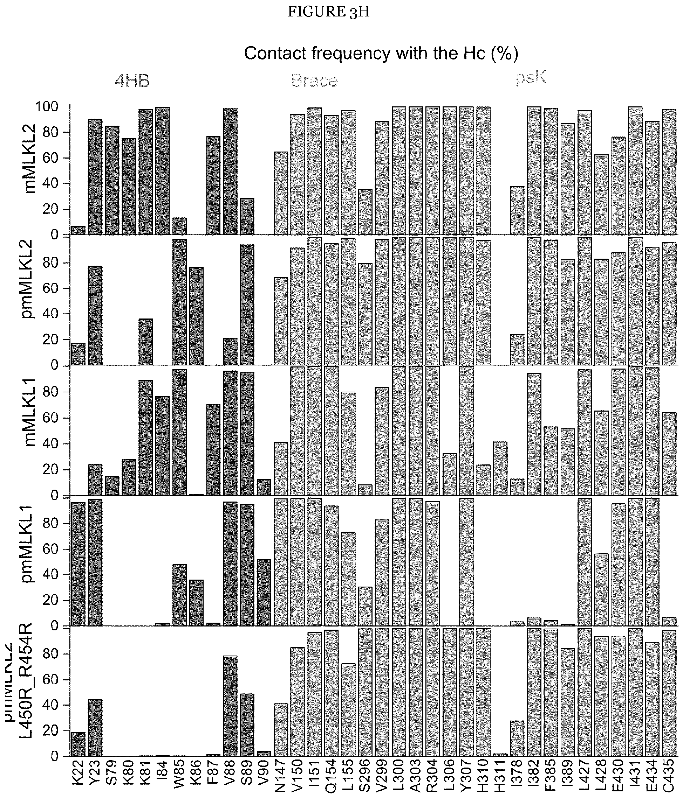

**Figure 3****:** shows that the accommodation of the Hc into a novel hydrophobic groove of mMLKL is required for necroptosis. A) 2D projections of PC1 and PC2 eigenvectors. PC1 describes the expansion of the psK domain coupled to the release of the 4HB domain and the rearrangement of the brace helices, while the PC2 describes the motions within the 4HB, the activation loop, and the loops connecting the 4HB, the brace region and the psK domain. B) Representative PC1 structures. More stable structures are represented in dark blue, while similar conformation states are represented in grey and light blue. Arrows indicate the direction of the movement. P indicates phosphorylation. C) Structural representation of the Hc and the hydrophobic groove of mMLKL2 and mMLKL1. Box: MLKL regions connected by the Hc. D) Structural representation of the Hc/groove of mMLKL2_450R_S454R E) 2D projections of the mutant. F-G) Necroptotic activity of the mutant. NIH-3T3 MLKL ko cells were transfected with the different variants of mMLKL-GFP and TSZ-treated. F) Dead cell population was quantified by flow cytometry from transfected cells. Individual values from at least three independent experiments are shown. G) Images from confocal microscopy. Pictures are representative of at least three independent experiments. Scale bar: 20 µm. H) Frequency of contacts of the Hc with different regions of MLKL structure, calculated as average obtained over the whole simulation.

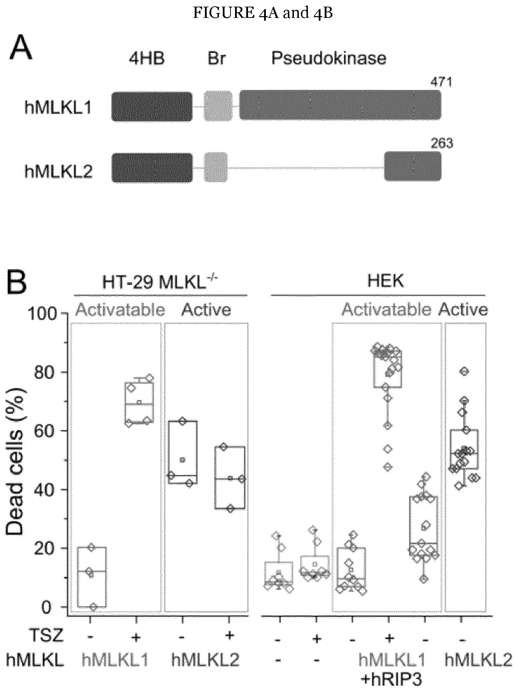

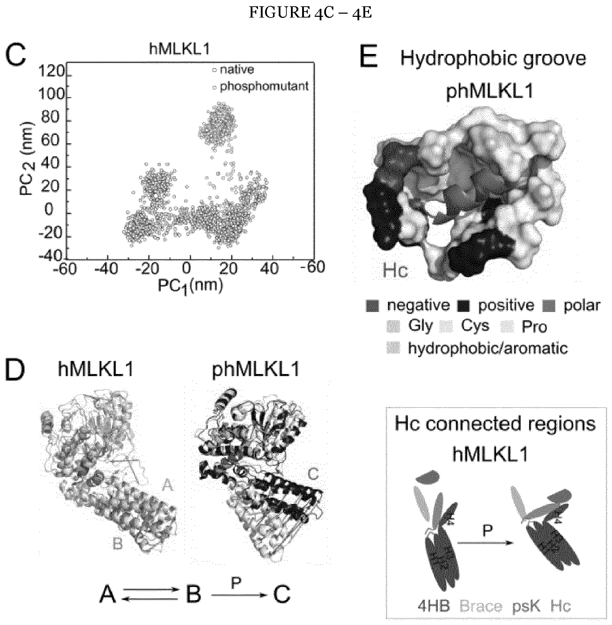

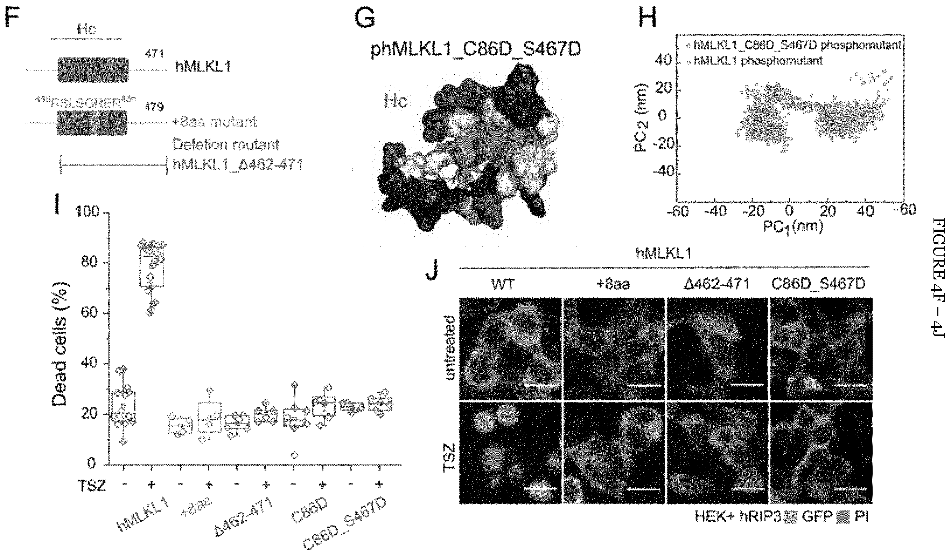

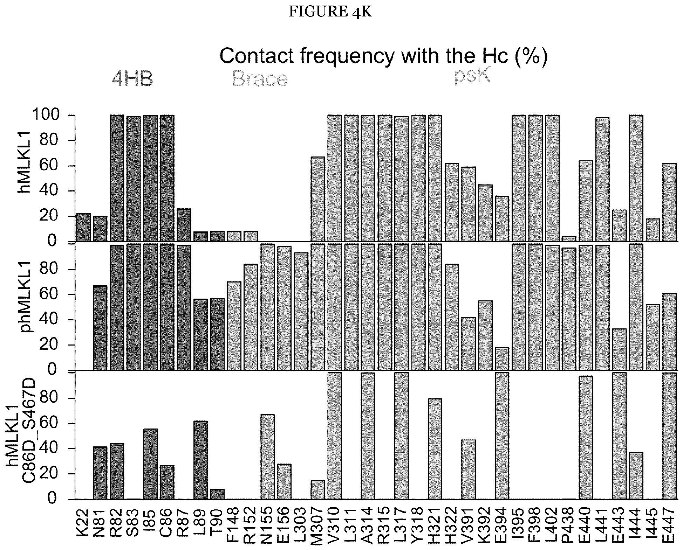

**Figure 4****:** shows that the activation of hMLKL also requires stabilization of the Hc/groove. A) Representation of human MLKL isoforms. B) Necroptotic activity of different isoforms of hMLKL. HT-29 MLKL ko or HEK cells transfected with one of the isoforms and treated with TSZ. MLKL ko cells were transfected with the different variants of mMLKL-GFP and HEK cells were additionally transfected (as indicated) with hRIP3. C) 2D projections of PC1 and PC2 eigenvectors. Arrows indicate the direction of the movement. D) Representative PC1 structures. The most stable conformation is represented in dark blue, while similar conformation states are represented in grey and light blue. P indicates phosphorylation. E) Structural representation of the Hc and the hydrophobic groove of hMLKLi. Box: MLKL regions connected by the Hc. F) Representation of insertion and deletion mutants of hMLKLi. G) Structural representation of the Hc/groove of hMLKL1_C86D_S467D. H) 2D projections of the mutant. I-J) Necroptotic activity of the mutants. HEK-RIP3 cells were transfected with the different variants of hMLKLi-GFP and TSZ-treated. I) The dead cell population was quantified by flow cytometry from transfected cells. Individual values from at least three independent experiments are shown. J) Images from confocal microscopy. Pictures are representative of at least three independent experiments. Scale bar: 20 µm. K) Frequency of contacts of the Hc with different regions of MLKL structure, calculated as average obtained over the whole simulation.

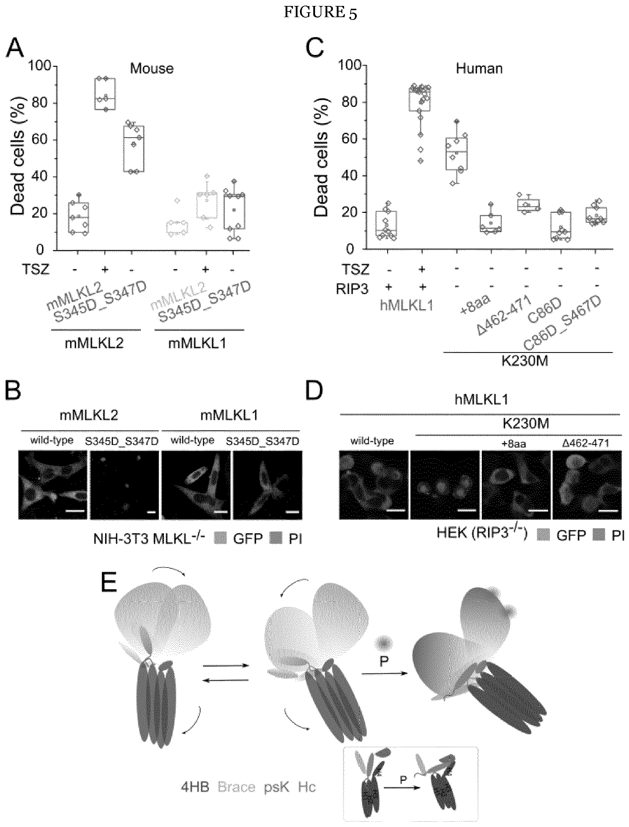

**Figure 5**: shows that the MLKL conformational switch induced by phosphorylation is blocked by alterations in the Hc/groove. A-D) Necroptotic activity of (A-B) mouse MLKL phosphomutants S345D_S347D or (C-D) human MLKL1_K230M. A and C) Flow cytometry measurements. B and D) Images from confocal microscopy. Cells (NIH MLKL ko for mouse and HEK for human) were transfected with the different variants of MLKL-GFP. The dead cell population was calculated from transfected cells. Individual values from at least three independent experiments are shown. Pictures are representative of at least three independent experiments. Scale bar: 20 µm. E) Model of MLKL auto-activation. Native MLKL fluctuates among different conformations in solution. Phosphorylation stabilizes the active conformation of activatable MLKL through specific interactions that are mediated by the Hc, which is accommodated in the hydrophobic groove. Correct accommodation of Hc into the hydrophobic groove is essential to transmit the conformational switch induced by phosphorylation to other regions of MLKL structure.

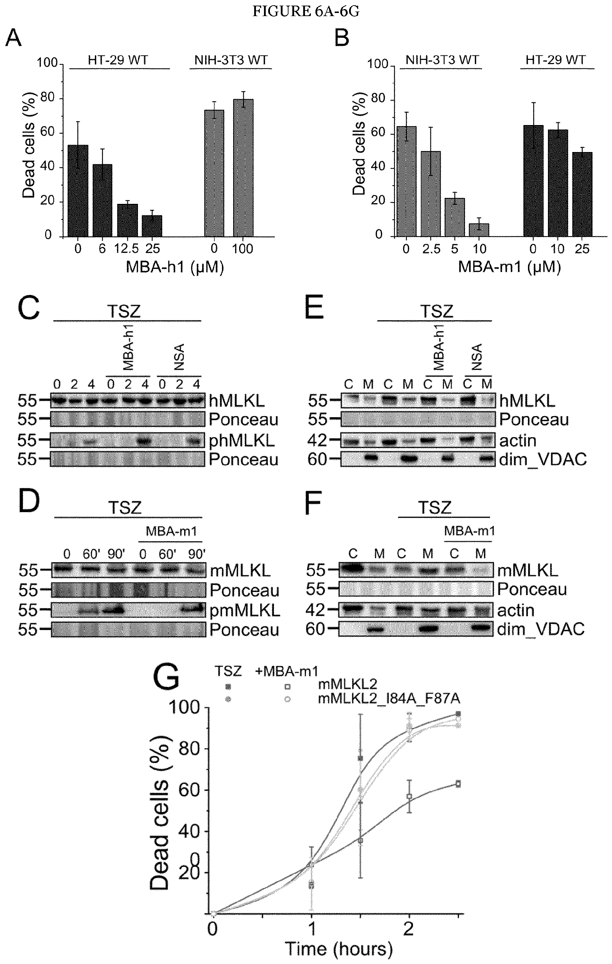

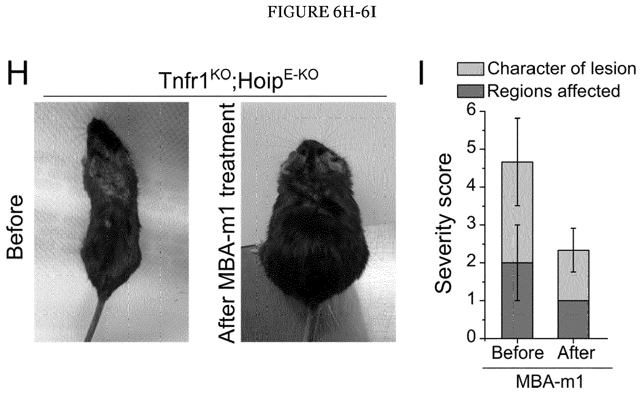

**Figure 6****:** shows that MBA-hi and MBA-mi are new specific inhibitors of human and mouse MLKL that act by blocking the Hc/groove. A-B) Dose-dependence of the inhibitory effect of MBA-hi (A) or MBA-miM (B) on necroptotic cell death in human (HT-29) and mouse (NIH-3T3) cells. C-D) Effect of MBA-hi (C) or MBA-mi (D) on MLKL phosphorylation. E-F) Effect of MBA-hi (E) or MBA-mi (F) on MLKL translocation to the plasma membrane. HT-29 (C and E) or NIH-3T3 (D and F) wt cells were TSZ-treated in the presence or not of the inhibitors. NSA was used as a control in C and E. G) Inhibitory effect of MBA-mi in MLKL ko cells transfected with mMLKL2 or the mutant mMLKL2_I84A_F87A. Cells were transfected with the different variants of MLKL-GFP. The dead cell population was calculated from transfected cells. Individual values from at least three independent experiments are shown. H-I) MBA-mi ameliorates dermatitis in Tnfri-KO HoipE-KO mice. H) Representative images of mice before and after 2 weeks' treatment with MBA-mi. I) Severity score of dermatitis assessed after and before treatment with the inhibitor. Tnfri-KO HoipE-KO (n=3). To quantify the sore of region affected, 0 values were assigned to no lesion, and 1 values were assigned when lesions were found in either neck, back, flank or head, being the sum of these elements taken as the final score. To quantify the character of lesions, 0 values were assigned to no lesions, 1 to excoriations one or small punctuate crust, 2 to multiple punctuate crust or coalescing crust, 3 to erosion, ulceration or bleeding. Data are presented as mean values ± s.e.m.

The sequences show:

SEQ ID NOs. 1 – mouse MLKL isoform 1

10 20 30 40 50
MDKLGQIIKL GQLIYEQCEK MKYCRKQCQR LGNRVHGLLQ PLQRLQAQGK
60 70 80 90 100
KNLPDDITAA LGRFDEVLKE ANQQIEKFSK KSHIWKFVSV GNDKILFHEV
110 120 130 140 150
NEKLRDVWEE LLLLLQVYHW NTVSDVSQPA SWQQEDRQDA EEDGNENMKV
160 170 180 190 200
ILMQLQISVE EINKTLKQCS LKPTQEIPQD LQIKEIPKEH LGPPWTKLKT
210 220 230 240 250
SKMSTIYRGE YHRSPVTIKV FNNPQAESVG IVRFTFNDEI KTMKKFDSPN
260 270 280 290 300
ILRIFGICID QTVKPPEFSI VMEYCELGTL RELLDREKDL TMSVRSLLVL
310 320 330 340 350
RAARGLYRLH HSETLHRNIS SSSFLVAGGY QVKLAGFELS KTQNSISRTA
360 370 380 390 400
KSTKAERSSS TIYVSPERLK NPFCLYDIKA EIYSFGIVLW EIATGKIPFE
410 420 430 440 450
GCDSKKIREL VAEDKKQEPV GQDCPELLRE IINECRAHEP SQRPSVDGRS
460 470
LSGRERILER LSAVEESTDK KV

SEQ ID NOs. 2 – mouse MLKL isoform 2

10 20 30 40 50
MDKLGQIIKL GQLIYEQCEK MKYCRKQCQR LGNRVHGLLQ PLQRLQAQGK
60 70 80 90 100
KNLPDDITAA LGRFDEVLKE ANQQIEKFSK KSHIWKFVSV GNDKILFHEV
110 120 130 140 150
NEKLRDVWEE LLLLLQVYHW NTVSDVSQPA SWQQEDRQDA EEDGNENMKV
160 170 180 190 200
ILMQLQISVE EINKTLKQCS LKPTQEIPQD LQIKEIPKEH LGPPWTKLKT
210 220 230 240 250
SKMSTIYRGE YHRSPVTIKV FNNPQAESVG IVRFTFNDEI KTMKKFDSPN
260 270 280 290 300
ILRIFGICID QTVKPPEFSI VMEYCELGTL RELLDREKDL TMSVRSLLVL
310 320 330 340 350
RAARGLYRLH HSETLHRNIS SSSFLVAGGY QVKLAGFELS KTQNSISRTA
360 370 380 390 400
KSTKAERSSS TIYVSPERLK NPFCLYDIKA EIYSFGIVLW EIATGKIPFE
410 420 430 440 450
GCDSKKIREL VAEDKKQEPV GQDCPELLRE IINECRAHEP SQRPSVDGIL
460
ERLSAVEEST DKKV

SEQ ID NOs. 3 – human MLKL isoform 1

10 20 30 40 50

60 70 80 90 100

110 120 130 140 150

160 170 180 190 200

210 220 230 240 250

260 270 280 290 300

310 320 330 340 350

360 370 380 390 400

410 420 430 440 450

460 470


SEQ ID NOs. 4 – human MLKL isoform 2

10          20          30          40          50
MENLKHIITL GQVIHKRCEE MKYCKKQCRR LGHRVLGLIK PLEMLQDQGK
60          70          80          90          100
RSVPSEKLTT AMNRFKAALE EANGEIEKFS NRSNICRFLT ASQDKILFKD
110         120         130         140         150
VNRKLSDVWK ELSLLLQVEQ RMPVSPISQG ASWAQEDQQD ADEDRRAFQM
160         170         180         190         200
LRRDNEKIEA SLRRLEINMK EIKETLRQSL ESSSGKSPLE ISRFKVKNVK
210         220         230         240         250
TGSASGCNSE KIRKLVAVKR QQEPLGEDCP SELREIIDEC RAHDPSVRPS
260
VDEILKKLST FSK

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Mouse MLKL isoforms have markedly different capabilities to mediate necroptosis

Mouse MLKL has three transcript variants produced by alternative splicing (Figure 1A). Variant 1 is annotated as the canonical form of MLKL (Uniprot database, Q9D2Y4-1). This transcript is the longest, encoding a protein of 472 amino acid residues (mMLKLi), whereas variant 2 is slightly shorter with 464 residues (mMLKL2). These two isoforms only differ in a sequence of eight amino acids, RSLSGRER, which is located at the C-terminal helix (Hc) of the psK domain of mMLKL1. There is additionally a variant 3 (mMLKL3) that encodes a shorter isoform that lacks the psK domain but contains a short extra sequence at the N-terminus. All three isoforms are generated by alternative 5' donor sites or exon-skipping alternative splicing mechanisms.

To evaluate the necroptotic potential of the different mMLKL isoforms, each of the three isoforms were re-expressed in NIH-3T3 MLKL knock out (ko) cells following induction of necroptosis with a mixture of TNF, a Smac mimetic compound (LCL-161) and the pan-caspase inhibitor zVAD (TSZ), which is the most commonly used necroptosis-inducing cocktail. The inventors quantified cell death by flow cytometry (Figure 1B) and confocal microscopy (Figure 1C) using propidium iodide (PI) as a marker of irreversible plasma membrane disruption. As expected, mMLKL3 was intrinsically active, on account of its lack of the psK domain. It had lower activity compared to the 4HB domain alone and was similar to the 4HB+brace mutant, in line with the inhibitory role attributed to the brace region (32, 33).

Surprisingly, mMLKL1 and mMLKL2 differed markedly in their capacity to induce necroptosis. Whereas mMLKL1 remained inactive following a stimulus that would normally trigger necroptosis, cells expressing mMLKL2 responded to treatment at the same levels as wild-type (wt), non-transfected NIH-3T3 cells. Specific expression of mMLKL2 in MLKL-deficient cells induced the typical necroptotic phenotype upon treatment: rounding up and detachment prior to plasma membrane breakdown. Similar results were obtained through the activation of the TNF pathway in L929 cells and in MLKL-deficient murine dermal fibroblasts (MLKL-ko MDFs) (not shown) or when necroptosis was triggered via TLR3/4 or DAI (Figure 1D). Also, these rather different activities of mMLKL1 and mMLKL2 were not the consequence of differential protein expression levels (not shown). Together, these results show that distinct MLKL isoforms have very different necroptosis-inducing potential with mMLKL1 being inactive, mMLKL2 being activatable and mMLKL3 being constitutively active (Figure 1).

The stark difference in necroptotic activity despite the high sequence similarity between mMLKL1 and mMLKL2 raised the question about their distribution in nature. To examine the expression levels of mMLKL1 and mMLKL2 in different tissues and cell lines from mouse, the inventors performed quantitative RT-PCR, with primers that specifically detect the RNA of mMLKL1 or mMLKL2. It was found that necroptosis-inactive mMLKL1 is expressed in the heart, liver, kidney and cecum, as well as in bone-marrow-derived macrophages (BMDMs) and NIH-3T3 cells (Figure 1E and F). mMLKL1 RNA represented a small fraction compared to the amount obtained for mMLKL2 in most of the cell lines and tissues except in macrophages, and neither isoform was detected in the negative control samples from MLKL ko mice (Figure 1E).

### Example 2: The C-terminal helix (Hc) is a key regulatory element for MLKL activation

The finding that the presence of eight additional amino acids in mMLKL1, as compared to mMLKL2, completely abolishes the capacity of MLKL to induce necroptosis implied that this segment could be important for MLKL regulation. To guide the experimental design for testing the structure-to-function relationship of these extra residues located before the very last C-terminal helix (Hc), molecular dynamics (MD) simulations were used. The inventors built 3D models of mMLKL1 and mMLKL2 based on the crystal structure of full mouse MLKL2 (pdb: 4btf) (21). First, filled gaps were refined in missing regions connecting different secondary structure elements within the 4HB (S79-K94), brace (Y118-Q128), and psK (K351-S358 and V456-V464) domains of the full mMLKL2 structure, since the absence of structural information in the crystal about these segments could mask important interactions of the C-terminus with other protein regions. The segment S79-K94 was modeled based on the α-helix 4 of the human 4HB domain (pdb code: 2msv) (33), while the other unsolved regions were modeled ab initio. Next, the 3D structure of mMLKL1 was modeled based on the refined structure of mMLKL2. Considering that MLKL is activated upon phosphorylation by RIP3, the inventors also modeled phosphomimetic versions of mMLKL1 and mMLKL2 (pmMLKLi and pmMLKL2) by mutating specific positions within the psK domain (S228E, S345D, S347D, T349D, and S352D) (34, 35). These residues are located opposite to the C-terminal segment of MLKL, in the N-lobe of the psK domain (Figure 2A and B).

Comparative analysis of the structures of mMLKL1 and mMLKL2 phosphomutants predicted that the Hc of inactive mMLKL1, but not that of mMLKL2, is unfolded due to the presence of the extra eight amino acids. This suggested that the loss of secondary structure in Hc could be linked, perhaps even causally, to the lack of necroptosis activity of mMLKL1.

To assess experimentally whether the effect of this insertion was sequence-specific, different mutants were produced in which Ala-segments of different lengths (2, 4, 6, and 8 Ala) were inserted into mMLKL2 at the equivalent position of the extra eight amino acid sequence of mMLKL1 (Figure 2C-G). Interestingly, insertion of even the smallest segment of two Ala caused the inactivation of mMLKL2, indicating that the insertion of any sequence before Hc abolishes mMLKL necroptotic activity (Figure 2D and G).

A previous study suggested that Hc could act as a suppressor of human MLKL activity by interacting with the α-helix 4 of the 4HB (36). To explore this possibility, the necroptotic activity of Hc deletion mutants was tested (Figure 2E). Deletion of the disordered C-terminal tail (Δ457-464) did not impact mMLKL2 activity. Unexpectedly, however, deletion of the Hc plus the C-terminal tail (Δ448-464) rendered mMLKL2 completely inactive (Figure 2F and G). These findings support a key role of Hc in MLKL regulation and imply that, in contrast to previous assumptions (36), Hc fulfills an activating rather than inhibitory role.

### Example 3: Accommodation of Hc into a novel hydrophobic groove is required for mMLKL activity

To predict the conformational changes that activate mMLKL2 upon phosphorylation, the inventors compared the MD simulations of mMLKL2 with its phosphomimetic version. To understand how the presence of the eight additional amino acids alters the structural dynamics of the protein, the inventors analyzed this for mMLKL1. An essential dynamics analysis was performed over the structural ensembles obtained through MD simulations. This method allows to predict differences in conformational dynamics among MLKL isoforms and the mutant structures in solution, which cannot be obtained from crystal structures (Amadei et al., 1993). As shown in Figure 3A and B top panels, the 2D projections of the principal components (PCi) and (PC2) suggested that non-phosphorylated mMLKL2 sampled two equally populated conformational states (denoted as A and B). mMLKL2 phosphorylation stabilized a more compact structure with a significantly reduced conformational subspace (90% of the simulation time remained at the stable state C) (Figure 3A). The presence of the eight extra residues in mMLKL1 restricted its conformational flexibility and prevented the stabilization of a dominant structure in the phosphomutant (Figure 3A and B bottom panels). Together, these MD simulations suggested that non-phosphorylated mMLKL1 and mMLKL2 fluctuate between conformational states (Figure 3A and B). Whereas phosphorylation seemed to stabilize a more compact conformation of mMLKL2, it had no notable effect on the structural dynamics of mMLKL1 (Figure 3A and B).

Inspection of the structure of mMLKL2 predicted that Hc is accommodated into a hydrophobic groove (Figure 3C top panel) connecting the Hc with residues from the 4HB, the brace region and the psK domain. Interestingly, this groove was disrupted in the model of mMLKL1, as indicated by the decrease in the contact frequency of its Hc with the 4HB and the psK (Figure 3C bottom panel). In the MD simulations, the Hc/groove of mMLKL2 underwent larger structural reorganizations upon activation when compared to mMLKL1. mMLKL2 activation induced readjustments in the interactions between the Hc and the 4HB and psK domains within the hydrophobic groove (Figure 3C) and decreased the stability of the brace helices. In contrast, a different set of interactions was established in the Hc/groove of mMLKL1, probably leading to an inactive conformation (Figure 3C box).

To test the hypothesis that Hc/groove association is essential for mMLKL2 activation, the double-mutant mMLKL2_L450R_S454R was designed. MD simulations predicted that replacement of Leu and Ser by the positively charged Arg would disrupt the Hc, thereby destabilizing its insertion into the groove and affecting the active conformation of mMLKL2 (Figure 3D and E). Accordingly, the inventors confirmed experimentally that mMLKL2_L450R_S454R was inactive when re-expressed in mMLKL-deficient cells treated with TSZ (Figure 3F and G). As before, the lack of activity of mMLKL2_L450R_S454R was neither attributable to an effect of the mutations on the protein expression level nor due to impairment of mMLKL phosphorylation (not shown). These results provided both the experimental validation for the predictions the inventors made about the essential role of the Hc/groove interaction for the necroptosis-inducing capacity of MLKL, and a satisfactory mechanistic explanation for the marked differences in this capacity between the mMLKL1 and mMLKL2 isoforms.

### Example 4: The Hc/groove interaction is also essential for activation of human MLKL

Human MLKL is annotated as two isoforms (hMLKLi and hMLKL2) that share the N-terminal and the C-terminal sequences, although hMLKL2 lacks a major part of the psK domain (Figure 4A). An isoform analogous to mMLKL1 has not been identified yet in human or any other organism. The functional characterization of these two human isoforms in MLKL ko HT-29 and HEK cells showed that hMLKLi could be activated via RIP3 phosphorylation upon exposure to necroptosis stimuli while hMLKL2 was intrinsically active (Figure 4B), as previously reported (36). Therefore, these two isoforms were functionally homologous to the mMLKL2 and mMLKL3 isoforms (Figure 1), with hMLKLi being activatable and hMLKL2 intrinsically active.

Human and mouse MLKL orthologues strongly differ in their psK domain (22) and in the underlying mechanism that controls their regulation (32, 37). In fact, both orthologues are unable to induce necroptosis when their host cells are exchanged (24), which raised the question whether Hc would also be relevant for hMLKL activity. The inventors used MD simulations to generate a structural model of activatable hMLKLi based on the NMR structure of the human 4HB domain (pdb: 2msv) (33), the crystal structure of its psK (pdb: 4mwi) (22), and the crystal structure of full length mouse MLKL (pdb: 4btf) (21). Phosphomimetic versions of human MLKL1 (phMLKLi) contained the mutations T357E and S358D (38). The MD simulations predicted that human MLKL1 sampled at least two conformational states (denoted as A and B), which were restricted to a single, different conformation in the phosphomutant (C) (Figure 4C and D), similar to activatable mMLKL2. Furthermore, Hc of hMLKL1 was also accommodated in the hydrophobic groove (Figure 4E). The temporal evolution of the structure suggested the formation of relevant interactions connecting Hc with 4HB, the brace region and the psK domain, thereby leading to increased compactness as well as Hc/groove and brace stabilization (Figure S4C-H).

The role of Hc in hMLKL1 activity was explored experimentally by assessing the necroptosis potential of different mutants (Figure 4F-J). Either the insertion of the eight amino acid sequence of mMLKL1 or the deletion of a C-terminal segment including the Hc completely abrogated the activity of hMLKLi (Figure 4F, I and J). Conversely, these mutations did not alter the intrinsic activity of hMLKL2. These results demonstrate that the Hc is also essential for hMLKLi, but not for hMLKL2 activity.

Cys86 was identified as a residue positioned at the core of the hydrophobic groove of hMLKLi in the region comprising the α-helix 4 of the 4HB. The inventors evaluated the effect of exchanging Cys86 (either alone or together with Ser467 in the Hc) by negatively charged Asp (Figure 4G-J). In the MD simulations, the Hc/groove interaction in hMLKLi was disrupted in the resulting mutant hMLKL1_C86D_S467D (Figure 4G), which had a detrimental impact on its conformational confinement and brace stability (Figure 4H and S4H). This effect was less dramatic in the simulations of hMLKL1_C86D (data not shown), which may require longer simulation time to destabilize the Hc/groove. In agreement with the MD predictions, experimentally it was found that the necroptotic activity of hMLKLi was abolished in both mutants. The defective function of hMLKL1_C86D and hMLKL1_C86D_S467D was not a result of differential hMLKL expression levels or phosphorylation. These results confirmed that, despite the inter-species structural differences, the essential role of the Hc/groove interaction in MLKL regulation is conserved between the murine and human orthologues.

### Example 5: Hc/groove coordinates MLKL activation downstream of phosphorylation

We next sought to discern the hierarchy between the Hc/groove interaction and MLKL phosphorylation in the process of activation. The inventors evaluated experimentally the activity of phosphomimetic variants of mMLKL1 and mMLKL2 generated by substitution of Ser345 and Ser347 by Asp residues (Figure 5A and B) (35). While introduction of these mutations in mMLKL2 induced stimulus-independent cell death, as expected from previous reports (35), mMLKL1 bearing the same mutations remained incapable of inducing necroptosis. As for the human homologue (hMLKLi), the inventors tested the intrinsically active mutant hMLKL1_K230M harboring a mutation that is located within the ATP-binding pocket imposing the conformational change attained upon phosphorylation (22). For this intrinsically-active mutant, the inventors assessed the effect of (i) the insertion of the extra eight amino acids sequence found in mMLKL1, (ii) the deletion of the C-terminal segment, and (iii) mutations in the Hc/groove (Figure 5C and D). Remarkably, all these modifications completely abolished the activity of hMLKL1_K230M, in line with the results obtained with mouse MLKL. Together, these findings demonstrate that altering the Hc/groove integrity interferes with the molecular switch that is driven by phosphorylation (Figure 5E) and required for necroptosis induction by both, human and mouse MLKL.

### Example 6: The Hc/groove interaction of MLKL can be selectively targeted by small molecules

Based on the conserved relevance of the Hc/groove for MLKL function, the inventors predicted that it should be possible to find small chemical compounds that inhibit MLKL activity by disrupting the Hc/groove interaction. Indeed, the structural analysis provided here for the Hc/groove of activatable MLKL variants affords the ability to rationally screen for and optimize such necroptosis-inhibiting small compounds.

By screening the "Tres Cantos Antimalarial Set" (TCAMS) publicly available at the Chembl-NTD database (http://www.ebi.ac.uk/chemblntd) with the Autodock Vina software, the inventors identified putative blockers that target the Hc/groove of hMLKLi or mMLKL2. Based on the scoring function of this initial analysis, the inventors selected the best-ranked commercially available compounds for human and mouse MLKL and tested them in necroptosis inhibition assays.

Thereby, the inventors identified two compounds, MBA-hi and MBA-mi, that specifically inhibited necroptosis in human HT-29 (MBA-h1) and mouse NIH-3T3 WT cells (MBA-mi), respectively, in a dose-dependent manner (Figure 6A and B and figure S6A and B). The species-specificity was expected considering the large difference in the Hc/groove interactions between mouse and human MLKL (Figure 3 and 4). MBA-hi inhibited necroptosis in HT-29 MLKL ko re-expressing hMLKLi, whereas MBA-mi had the same effect in NIH MLKL ko cells containing mMLKL2 (Figure S6C and D). These results confirm that both compounds act by inhibiting the activatable variants of human and mouse MLKL. The inhibitory activity of MBA-h1 was observed at concentrations lower than 10 µM, without evidence of toxicity (Figure 6A and figure S6E). MBA-mi was also able to inhibit necroptosis at similar concentrations but presented toxicity at higher concentrations (12.5-50 µM) (Figure 6B and figure S6F).

To further characterize the mechanism by which MBA-hi and MBA-mi inhibit the necroptosis-inducing capacity of MLKL, the inventors assessed whether they affect MLKL phosphorylation and plasma membrane translocation, two hallmarks of MLKL activation. Western-blot analysis with antibodies against phosphorylated MLKL showed that none of the compounds blocked the phosphorylation of MLKL, implying they indeed act by solely blocking MLKL without affecting the upstream kinases RIP1 and RIP3 (Figure 6C and D). Instead, both inhibitors potently interfered with MLKL translocation to the plasma membrane, similarly to NSA (Figure 6E and F). Together, these findings demonstrate that disrupting Hc/groove interactions with small molecules is a promising strategy to develop selective necroptosis inhibitors based on MLKL targeting.

As MBA-mi is the first specific inhibitor of mouse MLKL the inventors are aware of, the inventors sought to characterize its mechanism of inhibition in more detail. To obtain further evidence that MBA-mi inhibits necroptosis by specifically targeting the Hc/groove of mMLKL2, the inventors took a rational approach to design mutations that affect the MBA-mi/MLKL interaction without altering the necroptosis-inducing capacity of mMLKL2. In cell death assays, the inventors confirmed that the mutant mMLKL2_I84A_F87A was as active as mMLKL2, but that it was inert to inhibition by MBA-mi (Figure 6G). This result experimentally validates the proposed specific binding mode of MBA-mi to the Hc/groove of mMLKL2.

### Example 7: MBA-m1 ameliorates dermatitis in necroptosis-driven inflammatory skin disease

Finally, the inventors aimed to assess the efficacy of inhibiting necroptosis in vivo by specifically targeting the Hc/groove interaction of MLKL with MBA-mi in a mouse model with proven necroptosis contribution to disease. the inventors recently showed that mice in which the keratinocyte-specific absence of the LUBAC component HOIP (Hoip^{E-KO}) is combined with constitutive deficiency for TNFR1 (Tnfr1^{KO}) suffer from fatal skin inflammation at around day 70 after birth. This phenotype is substantially alleviated by constitutive deficiency in MLKL, showing that MLKL-dependent necroptosis contributes to the pathology (10). At around 30 to 40 days of age, the inventors treated *Tnfr1KO;HoipEKO* mice, which at this time suffer from mild dermatitis, with MBA-mi daily for the next three weeks. Strikingly, this treatment significantly ameliorated dermatitis as assessed by a decrease in the severity score of skin lesions at the end of treatment (Figure 6H and I). These results provide evidence that inhibiting MLKL activity by interfering with the Hc/groove interaction, in this case with the small molecule MBA-mi, is possible in vivo and that this treatment prevents the progression of a necroptosis-driven pathology (10)

### REFERENCES

The references are:
1. T. Vanden Berghe, A. Linkermann, S. Jouan-Lanhouet, H. Walczak, P. Vandenabeele, Regulated necrosis: the expanding network of non-apoptotic cell death pathways. Nature reviews. Molecular cell biology 15, 135 (Feb, 2014).
2. D. Wallach, T. B. Kang, C. P. Dillon, D. R. Green, Programmed necrosis in inflammation: Toward identification of the effector molecules. Science 352, aaf2154 (Apr 1, 2016).
3. Z. Su, Z. Yang, L. Xie, J. P. DeWitt, Y. Chen, Cancer therapy in the necroptosis era. Cell death and differentiation 23, 748 (May, 2016).
4. M. Seehawer et al., Necroptosis microenvironment directs lineage commitment in liver cancer. Nature 562, 69 (Oct, 2018).
5. N. Yatim, S. Cullen, M. L. Albert, Dying cells actively regulate adaptive immune responses. Nature reviews. Immunology 17, 262 (Apr, 2017).
6. Z. Ying et al., Mixed Lineage Kinase Domain-like Protein MLKL Breaks Down Myelin following Nerve Injury. Molecular cell 72, 457 (Nov 1, 2018).
7. S. Zhang, M. B. Tang, H. Y. Luo, C. H. Shi, Y. M. Xu, Necroptosis in neurodegenerative diseases: a potential therapeutic target. Cell death & disease 8, e2905 (Jun 29, 2017).
8. W. J. Kaiser et al., Toll-like receptor 3-mediated necrosis via TRIF, RIP3, and MLKL. The Journal of biological chemistry 288, 31268 (Oct 25, 2013).
9. H. Nailwal, F. K. Chan, Necroptosis in anti-viral inflammation. Cell death and differentiation 26, 4 (Jan, 2019).
10. L. Taraborrelli et al., LUBAC prevents lethal dermatitis by inhibiting cell death induced by TNF, TRAIL and CD95L. Nature communications 9, 3910 (Sep 25, 2018).
11. E. J. Petrie, P. E. Czabotar, J. M. Murphy, The Structural Basis of Necroptotic Cell Death Signaling. Trends in biochemical sciences 44, 53 (Jan, 2019).
12. R. A. Espiritu, L. Pedrera, U. Ros, Tuning the way to die: implications of membrane perturbations in necroptosis. 29, 201 (2019).
13. L. Sun et al., Mixed lineage kinase domain-like protein mediates necrosis signaling downstream of RIP3 kinase. Cell 148, 213 (Jan 20, 2012).
14. J. Zhao et al., Mixed lineage kinase domain-like is a key receptor interacting protein 3 downstream component of TNF-induced necrosis. Proceedings of the National Academy of Sciences of the United States of America 109, 5322 (Apr 3, 2012).
15. M. C. de Almagro, D. Vucic, Necroptosis: Pathway diversity and characteristics. Seminars in cell & developmental biology 39, 56 (Mar, 2015).
16. E. J. Petrie, J. M. Hildebrand, J. M. Murphy, Insane in the membrane: a structural perspective of MLKL function in necroptosis. Immunology and cell biology 95, 152 (Feb, 2017).
17. Z. Cai et al., Plasma membrane translocation of trimerized MLKL protein is required for TNF-induced necroptosis. Nature cell biology 16, 55 (Jan, 2014).
18. X. Chen et al., Translocation of mixed lineage kinase domain-like protein to plasma membrane leads to necrotic cell death. Cell research 24, 105 (Jan, 2014).
19. Y. Dondelinger et al., MLKL compromises plasma membrane integrity by binding to phosphatidylinositol phosphates. Cell reports 7, 971 (May 22, 2014).
20. B. Xia et al., MLKL forms cation channels. Cell research 26, 517 (May, 2016).
21. J. M. Murphy et al., The pseudokinase MLKL mediates necroptosis via a molecular switch mechanism. Immunity 39, 443 (Sep 19, 2013).
22. J. M. Murphy et al., Insights into the evolution of divergent nucleotide-binding mechanisms among pseudokinases revealed by crystal structures of human and mouse MLKL. The Biochemical journal 457, 369 (Feb 1, 2014).
23. J. M. Hildebrand et al., Activation of the pseudokinase MLKL unleashes the four-helix bundle domain to induce membrane localization and necroptotic cell death. Proceedings of the National Academy of Sciences of the United States of America 111, 15072 (Oct 21, 2014).
24. M. C. Tanzer et al., Evolutionary divergence of the necroptosis effector MLKL. Cell death and differentiation 23, 1185 (Jul, 2016).
25. B. Yan et al., Discovery of a new class of highly potent necroptosis inhibitors targeting the mixed lineage kinase domain-like protein. Chemical communications 53, 3637 (Mar 28, 2017).
26. W. J. Kaiser et al., RIP3 mediates the embryonic lethality of caspase-8-deficient mice. Nature 471, 368 (Mar 17, 2011).
27. P. A. Harris et al., Discovery of Small Molecule RIP1 Kinase Inhibitors for the Treatment of Pathologies Associated with Necroptosis. ACS medicinal chemistry letters 4, 1238 (Dec 12, 2013).
28. P. A. Harris et al., Discovery of a First-in-Class Receptor Interacting Protein 1 (RIP1) Kinase Specific Clinical Candidate (GSK2982772) for the Treatment of Inflammatory Diseases. Journal of medicinal chemistry 60, 1247 (Feb 23, 2017).
29. T. Xie et al., Structural basis of RIP1 inhibition by necrostatins. Structure 21, 493 (Mar 5, 2013).
30. D. Ofengeim, J. Yuan, Regulation of RIP1 kinase signalling at the crossroads of inflammation and cell death. Nature reviews. Molecular cell biology 14, 727 (Nov, 2013).
31. W. Declercq, T. Vanden Berghe, P. Vandenabeele, RIP kinases at the crossroads of cell death and survival. Cell 138, 229 (Jul 23, 2009).
32. K. A. Davies et al., The brace helices of MLKL mediate interdomain communication and oligomerisation to regulate cell death by necroptosis. Cell death and differentiation 25, 1567 (Sep, 2018).
33. L. Su et al., A plug release mechanism for membrane permeation by MLKL. Structure 22, 1489 (Oct 7, 2014).
34. M. C. Tanzer et al., Necroptosis signalling is tuned by phosphorylation of MLKL residues outside the pseudokinase domain activation loop. The Biochemical journal 471, 255 (Oct 15, 2015).
35. D. A. Rodriguez et al., Characterization of RIPK3-mediated phosphorylation of the activation loop of MLKL during necroptosis. Cell death and differentiation 23, 76 (Jan, 2016).
36. K. H. Arnez et al., Analysis of the N-terminal region of human MLKL, as well as two distinct MLKL isoforms, reveals new insights into necroptotic cell death. Bioscience reports 36, e00291 (2016).
37. E. J. Petrie et al., Conformational switching of the pseudokinase domain promotes human MLKL tetramerization and cell death by necroptosis. Nature communications 9, 2422 (Jun 21, 2018).
38. H. Wang et al., Mixed lineage kinase domain-like protein MLKL causes necrotic membrane disruption upon phosphorylation by RIP3. Molecular cell 54, 133 (Apr 10, 2014).
39. E. H. Kim, S. W. Wong, J. Martinez, Programmed Necrosis and Disease:We interrupt your regular programming to bring you necroinflammation. Cell death and differentiation 26, 25 (Jan, 2019).
40. M. N. Messmer, A. G. Snyder, A. Oberst, Comparing the effects of different cell death programs in tumor progression and immunotherapy. Cell death and differentiation 26, 115 (Jan, 2019).
41. J. K. Rathkey et al., Chemical disruption of the pyroptotic pore-forming protein gasdermin D inhibits inflammatory cell death and sepsis. Science immunology 3, (Aug 24, 2018).
42. A. Sali, T. L. Blundell, Comparative protein modeling by satisfaction of spatial restraints. . J. Mol. Biol. 234, 779 (1993).
43. M. S. Madhusudhan, B. M. Webb, M. A. Marti-Renom, N. Eswar, A. Sali, Alignment of multiple protein structures based on sequence and structure features. Protein Eng Des Sel. 22, 569 (September 1, 2009, 2009).
44. B. J. Bender et al., Protocols for Molecular Modeling with Rosetta3 and RosettaScripts. Biochemistry 55, 4748 (Aug 30, 2016).
45. M. J. Abraham et al., GROMACS: High performance molecular simulations through multi-level parallelism from laptops to supercomputers. SoftwareX 1-2, 19 (2015/09/01/, 2015).
46. K. Lindorff-Larsen et al., Improved side-chain torsion potentials for the Amber ff99SB protein force field. Proteins 78, 1950 (2010).
47. L. J. William, C. Jayaraman, D. M. Jeffry, W. I. Roger, L. K. Michael. (AIP, 1983), vol. 79, pp. 926-935.
48. H. Mesa-Galloso et al., Disrupting a key hydrophobic pair in the oligomerization interface of the actinoporins impairs their pore-forming activity. Protein Sci 26, 550 (Mar, 2017).
49. A. Amadei, A. B. M. Linssen, H. J. C. Berendsen, Essential dynamics of proteins. Proteins: Structure, Function, and Bioinformatics 17, 412 (1993).
50. J. S. Hub, B. L. de Groot, Detection of functional modes in protein dynamics. PLoS Comput Biol 5, e1000480 (Aug, 2009).
51. O. Trott, A. J. Olson, AutoDock Vina: Improving the speed and accuracy of docking with a new scoring function, efficient optimization, and multithreading. J. Comput. Chem 31, 455 (2010).

## Claims

1. **A *non-therapeutic* method for modulating the activation capacity of necroptosis in a cell,** the method comprising the step of contacting the cell with a MLKL modulating compound or MLKL modulating composition, wherein said MLKL modulating compound or MLKL modulating composition when contacted with the cell modulates an intramolecular interaction between the C-terminal helix (Hc) of the psK domain and a hydrophobic groove, wherein the hydrophobic groove is a 3 dimensional structural motif in the MLKL protein, or in the variant MLKL protein, that comprises and/or involves at least one amino acid residue from each of the 4HB domain, the brace region and the psK domain.

2. The *non-therapeutic* method according to claim 1, wherein the Hc is located at the C-terminal end of the psK domain, and in the event the MLKL protein, or MLKL variant protein, is human MLKL1 (SEQ ID NO: 3), is between amino acids 400 to 471, preferably 450 to 471, most preferably between 460 to 471, most preferably is between amino acid 458 to 468; and in the event the MLKL protein, or MLKL variant protein, is mouse MLKL2 (SEQ ID NO: 2), is between amino acids 400 to 464, preferably 420 to 464, most preferably between 430 to 460, and most preferably is between amino acids 445 to 455.

3. The *non-therapeutic* method according to claim 1 or 2, wherein the hydrophobic groove wherein the hydrophobic groove comprises at least one amino acid residue from each of the 4HB domain, the brace region and the psK domain, and
(i) in the event the MLKL protein, or MLKL variant protein, is human MLKL1 (SEQ ID NO: 3), the at least one amino acid residue of the 4HB domain is selected from a sequence between amino acid 80 and 100, more preferably 80 to 90, and most preferably are amino acids 81, 82, 83, 85, 86, 87, 89 or 90 of human MLKL; the at least one amino acid residue of the brace region is selected from a sequence between amino acid 120 and 190, more preferably 140 to 160, and most preferably are amino acids 148, 152, 155 or 156 from human MLKL; and the at least one amino acid residue of the psK domain is selected from a sequence between amino acid 190 and 471, more preferably 300 to 450, and most preferably are amino acids 303, 307, 310, 311, 314, 315, 317, 318, 321, 322, 391, 392, 394, 395, 398, 402, 438, 440, 441, 443, 444, 445, 447 from human MLKL; or
(ii) in the event the MLKL protein, or MLKL variant protein, is mouse MLKL2 (SEQ ID NO: 2), the at least one amino acid residue of the 4HB domain is selected from a sequence between amino acid 20 and 100, more preferably 20 to 90, and most preferably are amino acids 23, 79, 80, 81, 84, 85, 86, 87, 88 or 89; the at least one amino acid residue of the brace region is selected from a sequence between amino acid 120 and 190, more preferably 140 to 160, and most preferably are amino acids 147, 150, 151, 154 or 155; the at least one amino acid residue of the psK domain is selected from a sequence between amino acid 190 and 464, more preferably 290 to 440, and most preferably are amino acids 296, 299, 300, 303, 304, 306, 307, 310, 311, 378, 382, 385, 389, 427, 428, 430, 431, 434 or 435.

4. **A *non-therapeutic* method for identifying a compound capable of modulating activation of MLKL,** the method comprising
(i) Bringing into contact an MLKL protein, or an MLKL variant protein, and/or a cell expressing an MLKL protein, or an MLKL variant protein, with a candidate compound, wherein the MLKL protein, or the MLKL variant protein, comprises at least an N-terminal four helix bundle (4HB) domain, a C-terminal pseudo kinase (psK) domain, connected by a brace region;
(ii) Optionally, activating the MLKL protein, or the MLKL variant protein;
(iii) Determining the intramolecular interaction between the C-terminal helix (Hc) of the psK domain and the hydrophobic groove as defined in claim 14, of the MLKL protein, or the MLKL variant protein, when contacted with the candidate compound;
wherein: (i) an altered intramolecular interaction between Hc and the hydrophobic groove of the MLKL protein, or the MLKL variant protein, contacted with the candidate compound compared to an MLKL protein, or the MLKL variant protein, not contacted with the candidate compound, and/or (ii) an altered intramolecular interaction between Hc and the hydrophobic groove of the MLKL protein, or the MLKL variant protein, expressed by a cell contacted with the candidate compound compared to a cell not contacted with the candidate compound; indicated that the candidate compound is a compound capable of modulating the activation of MLKL.

5. The *non-therapeutic* method according to claim 4, wherein the MLKL protein, or variant MLKL protein, is a constitutively active MLKL protein, for example a phosphorylated MLKL protein.

6. The *non-therapeutic* method according to claim 4, wherein step (ii) is mandatory and comprises phosphorylation of the MLKL protein, or the MLKL variant protein; and/or comprises inducing necroptosis in the cell expressing the MLKL protein, or MLKL variant protein, or alternatively comprises trafficking, pathological immune responses and/or inflammation.

7. **A *non-therapeutic* method for identifying a compound capable of modulating activation of MLKL**, the method comprising:
(i) Bringing into contact a MLKL Hc domain with a candidate compound,
(ii) Determining a specific binding of the candidate compound to the MLKL Hc domain,
Wherein a specific binding of the candidate compound to the MLKL Hc domain compared to the binding to an unrelated protein domain indicates that the candidate compound is capable of modulating activation of MLKL.

8. **A *non-therapeutic* method for identifying a compound capable of modulating activation of MLKL,** the method comprising:
(i) Bringing into contact a candidate compound with a MLKL hydrophobic groove domain which is a protein domain comprising amino acid residues from the 4HB domain, the brace region and the psK domain,
(ii) Determining a specific binding of the candidate compound to the MLKL hydrophobic groove domain,
wherein a specific binding of the candidate compound to the MLKL hydrophobic groove domain compared to the binding to an unrelated protein domain indicates that the candidate compound is capable of modulating activation of MLKL.

9. The *non-therapeutic* method according to claim 7 or 8, wherein the MLKL hydrophobic groove domain and/or the MLKL Hc domain are provided as full-length MLKL protein, or are provided as test proteins comprising the MLKL hydrophobic groove domain and/or the MLKL Hc domain, but not comprising the full psK and/or 4HB domain, more preferably the test protein is a MLKL mutant protein, such as a constitutively active MLKL protein, or a constitutively active MLKL variant protein.

10. **A compound or composition for use in the treatment of a disease in a subject,** wherein the treatment comprises modulating in the subject the activation of Mixed lineage kinase domain-like (MLKL) protein, or a MLKL variant protein, wherein the MLKL protein, or MLKL variant protein, comprises at least an N-terminal four helix bundle (4HB) domain, a C-terminal pseudo kinase (psK) domain, connected by a brace region, and wherein the method comprises the modulation of the intramolecular interaction between the C-terminal helix (Hc) of the psK domain and a hydrophobic groove, wherein the hydrophobic groove is a 3 dimensional structural motif in the MLKL protein, or in the variant MLKL protein, that comprises and/or involves at least one amino acid residue from each of the 4HB domain, the brace region and the psK domain.

11. The compound or composition for use according to claim 15, wherein the Hc is located at the C-terminal end of the psK domain, and in the event the MLKL protein, or MLKL variant protein, is human MLKL1 (SEQ ID NO: 3), is between amino acids 400 to 471, preferably 450 to 471, most preferably between 460 to 471, and most preferably is between amino acid 458 to 468; and in the event the MLKL protein, or MLKL variant protein, is mouse MLKL2 (SEQ ID NO: 2), is between amino acids 400 to 464, preferably 420 to 464, most preferably between 430 to 460, and most preferably is between amino acids 445 to 455.

12. The compound or composition for use according to claim 10 or 11, wherein the hydrophobic groove comprises at least one amino acid residue from each of the 4HB domain, the brace region and the psK domain, and
(i) in the event the MLKL protein, or MLKL variant protein, is human MLKL1 (SEQ ID NO: 3), the at least one amino acid residue of the 4HB domain is selected from a sequence between amino acid 80 and 100, more preferably 80 to 90, and most preferably are amino acids 81, 82, 83, 85, 86, 87, 89 or 90 of human MLKL; the at least one amino acid residue of the brace region is selected from a sequence between amino acid 120 and 190, more preferably 140 to 160, and most preferably are amino acids 148, 152, 155 or 156 from human MLKL; and the at least one amino acid residue of the psK domain is selected from a sequence between amino acid 190 and 471, more preferably 300 to 450, and most preferably are amino acids 303, 307, 310, 311, 314, 315, 317, 318, 321, 322, 391, 392, 394, 395, 398, 402, 438, 440, 441, 443, 444, 445, 447 from human MLKL; or
(ii) in the event the MLKL protein, or MLKL variant protein, is mouse MLKL2 (SEQ ID NO: 2), the at least one amino acid residue of the 4HB domain is selected from a sequence between amino acid 20 and 100, more preferably 20 to 90, and most preferably are amino acids 23, 79, 80, 81, 84, 85, 86, 87, 88 or 89; the at least one amino acid residue of the brace region is selected from a sequence between amino acid 120 and 190, more preferably 140 to 160, and most preferably are amino acids 147, 150, 151, 154 or 155; the at least one amino acid residue of the psK domain is selected from a sequence between amino acid 190 and 464, more preferably 290 to 440, and most preferably are amino acids 296, 299, 300, 303, 304, 306, 307, 310, 311, 378, 382, 385, 389, 427, 428, 430, 431, 434 or 435.

13. The compound or composition for use according to any one of claims 10 to 12, wherein the MLKL modulating compound or MLKL modulating composition is selected from a polypeptide, peptide, glycoprotein, a peptidomimetic, an antigen binding construct (for example, an antibody, antibody-like molecule or other antigen binding derivative, or an or antigen binding fragment thereof), a nucleic acid such as a DNA or RNA, for example an antisense or inhibitory DNA or RNA, a ribozyme, an RNA or DNA aptamer, RNAi, siRNA, shRNA and the like, including variants or derivatives thereof such as a peptide nucleic acid (PNA), a genetic construct for targeted gene editing, such as a CRISPR/Cas9 construct and/or a guide nucleic acid (gRNA or gDNA) and/or tracrRNA, or a small molecular compound, preferably a small molecule having a molecular weight of less than 5 kD, more preferably of less than 2kD, most preferably of less than 1kD, or any combination of the above.

14. The compound or composition for use according to any one of claims 10 to 13, wherein the compound or composition is any of the following compounds, as well as derivatives, and solvates, salts, stereoisomers, complexes, polymorphs, crystalline forms, racemic mixtures, diastereomers, enantiomers, tautomers, isotopically labelled forms, prodrugs, and combinations thereof:

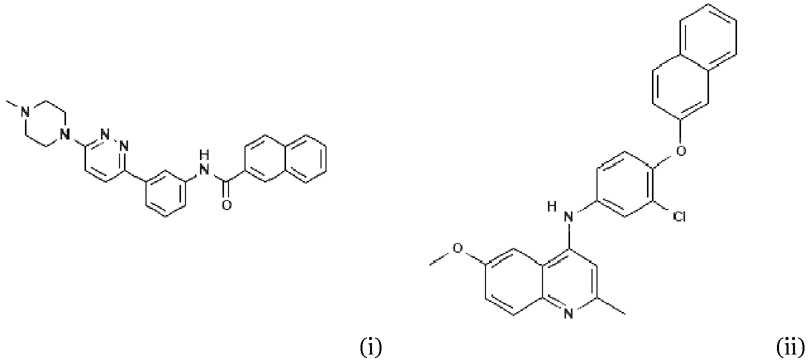

15. The compound or composition for use according to any one of claim 10 to 14, wherein the disease associated with necroptosis is selected from the group consisting of diseases of the bones, joints, connective tissue and cartilage, muscular diseases, skin diseases, cardiovascular diseases, circulatory diseases, hematological and vascular diseases, diseases of the lung, diseases of the gastro-intestinal tract, diseases of the liver, diseases of the pancreas, metabolic diseases, diseases of the kidneys, viral and bacterial infections, severe intoxications, degenerative diseases associated with the Acquired Immune Deficiency Syndrome (AIDS), disorders associated with aging, inflammatory diseases, auto-immune diseases, dental disorders, ophthalmic diseases or disorders, diseases of the audition tracts, diseases associated with mitochondria, and cancer, such as a solid cancer or lymphoid cancer, and cancer metastasis.
